# EUROPÄISCHE PATENTSCHRIFT

(11) **EP 0 959 070 B1**
(45) Veröffentlichungstag und Bekanntmachung des Hinweises auf die Patenterteilung: **12.09.2007**
(21) Anmeldenummer: 99109477.2
(22) Anmeldetag: 12.05.1999
(51) Int. Cl.: C07D 207/273, C07C 229/22, C07C 229/20, C07D 237/04

(54) **Verfahren zur Herstellung von 3-Amino-2-oxopyrrolidinen, neue Zwischenprodukte und deren Verwendung**
Process for the production of 3-amino-2-oxopyrrolidines, novel intermediates and their use
Procédé de préparation de 3-amino-2-oxopyrrolidines, nouveaux produits intermédiaires et leur utilisation

(30) Priorität: 22.05.1998 DE 19822912
(43) Veröffentlichungstag der Anmeldung: 24.11.1999
(73) Patentinhaber: Degussa GmbH, 40474 Düsseldorf (DE)
(72) Erfinder: Drauz, Karlheinz Prof., 63579 Freigericht (DE); Knaup, Günter Dr., 63486 Bruchköbel (DE); Schwarm, Michael Dr., 63755 Alzenau (DE)

(56) Entgegenhaltungen:
- JP-A- 49 016 109
- US-A- 4 012 519
- US-A- 4 460 603
- K. UNDHEIM, G. A. ULSAKER: "N-Quaternary Compounds" ACTA CHEM. SCAND., Bd. 27, Nr. 3, 1973, Seiten 1059-1066, XP002111925
- B. E. EVANS ET AL.: "Nanomolar-affinity, Non-Peptide Oxytocin Receptor antagonists" J. MED. CHEM., Bd. 36, Nr. 25, 1993, Seiten 3993-4005, XP002111926
- G. J. KOOMEN ET AL.: "Unconventional Nucleotide Analogues. Part X. Synthesis of N-Substituted 3-(Adenin-9-yl)pyrrolidin-2-ones" J. CHEM. SOC. PERKIN TRANS. 1,1973, Seiten 1934-1940, XP002111927
- Z. PROCHAZKA ET AL.: "Synthesis and some biological properties of analogue of angiotensin with modified proline structure" COLLECT. CZECH. CHEM. COMMUN., Bd. 55, Nr. 12, 1990, Seiten 3008-3014, XP002111928
- R. M. FREIDINGER ET AL.: "Protected Lactam-Bridged Dipeptides for Use as Conformational Constraints in Peptides" J. ORG. CHEM., Bd. 47, Nr. 1, 1982, Seiten 104-109, XP002111929
- G. CAVICCHIONI ET AL.: "Synthesis and Biological Activity of a Conformationally Constrained Chemotactic gamma-Lactam Formyl Tetrapeptide" ARZNEIM. FORSCH., Bd. 46, Nr. 10, 1996, Seiten 964-966, XP002111930
- M. J. DEAL ET AL.: "Conformationally Constrained Tachykinin Analogues: Potent and Highly Selective Neurokinin NK-2-Receptor Antagonists" J. MED. CHEM., Bd. 35, Nr. 22, 1992, Seiten 4195-4204, XP002111931
- K. BARLOS ET AL.: "Anwendung von N-Tritylmethionin zur Darstellung von biologisch und synthetisch interessanten Heterocyclen" LIEBIGS ANN. CHEM.,1988, Seiten 1127-1134, XP002111932
- J. E. SEMPLE ET AL.: "Design, Synthesis, and Evaluation of a Novel, Selective, and Orally Bioavailable Class of Thrombin Inhibitors: 1-Argininal Derivatives Incorporating P3-P4 Lactam Sulfonamide Moieties" J. MED. CHEM., Bd. 39, Nr. 23, 1996, Seiten 4531-4536, XP002081550
- B. J. WILLIAMS ET AL.: "Cyclic Peptides as Selective Tachykinin Antagonists" J. MED. CHEM., Bd. 36, Nr. 1, 1993, Seiten 2-10, XP002111933
- PATENT ABSTRACTS OF JAPAN vol. 012, no. 312, 24. August 1988 (1988-08-24) & JP 63 083082 A (TAKEDA CHEM. IND., LTD.), 13. April 1988 (1988-04-13)
- Y. KNOBLER ET AL.: "Lactam Formation through Aminolysis of alpha-Amino-gamma-butyrolacton. 2-Amino-4-hydroxybutyramides and 1-Aryl 3-Aminopyrrolidin-2-ones" J. ORG. CHEM., Bd. 29, Nr. 5, 1964, Seiten 1229-1236, XP002111934
- R. LALIBERT¹, L. BERLINGUET: "Synthèses d'acides aminobutyriques II. Acides alkylamino-2 hydroxy-4 butyriques" CAN. J. CHEM., Bd. 40, 1962, Seiten 1960-1964, XP002111935
- M. KAWAI ET AL.: "CD spectra of DNP derivatives of aromatic alpha-amino acids and related compounds" TETRAHEDRON, Bd. 34, 1978, Seiten 3435-3444, XP002111936
- S. P. L. SÖRENSEN, A. C. ANDERSEN: "Studien über Aminosäuresynthesen. VIII. Diaminodicarbonsäuren und Oxyaminosäuren" HOPPE-SEYLER'S Z. PHYSIOL. CHEM., Bd. 56, 1908, Seiten 275-304, XP002111937
- P. CHOCAT ET AL.: "Synthesis of Selenocystine and Selenohomocystine with O-Acetylhomoserine Sulfhydrylase" AGRIC. BIOL. CHEM., Bd. 49, Nr. 4, 1985, Seiten 1143-1150, XP002111938
- S. NAGAI, M. FLAVIN: "Acetylhomoserine" J. BIOL. CHEM., Bd. 242, Nr. 17, 1967, Seiten 3884-3895, XP002111939
- D. M. KALVIN, R. W. WOODARD: "Synthesis of (4R)-D,L-[4-(2)H]- and (4S)-[4-(2)H]Homoserine Lactones" J. ORG. CHEM., Bd. 50, Nr. 13, 1985, Seiten 2259-2263, XP002111940
- N. ESAKI ET AL.: "Reactions of O-Substituted L-Homoserines Catalyzed by L-Methionine gamma-Lyase and Their Mechanism" AGRIC. BIOL. CHEM., Bd. 48, Nr. 8, 1984, Seiten 1991-1996, XP002111941
- P. MALATESTA ET AL.: "alpha-n-butilammino-beta-cloropropio(gamm a-clorobutirro)anilidi ad attività anestetica locale" ANN. CHIM. (ROME), Bd. 55, 1965, Seiten 133-142, XP002111942
- R. SUDO ET AL.: "Studies on gamma-butyrolactone derivatives. I. Synthesis of alpha-amino-gamma-butyrolactone" NIPPON KAGAKU ZASSHI, Bd. 74, 1953, Seiten 1009-11013, XP002111943 & CHEMICAL ABSTRACTS, vol. 49, no. 10, 25. Mai 1955 (1955-05-25) Columbus, Ohio, US; abstract no. i, Spalte 6829;

## Beschreibung

Die vorliegende Erfindung betrifft ein Verfahren zur Herstellung von Verbindungen der allgemeinen Formel I und deren Salze 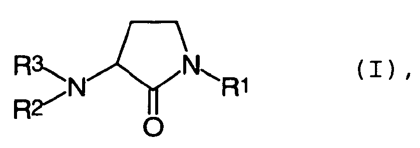 worin
R¹ bedeuten kann H, (C₁-C₈)-Alkyl, (C₂-C₈)-Alkenyl, (C₂-C₈)-Alkinyl, (C₂-C₈)-Alkoxyalkyl, (C₁-C₈)-Acyl, welche gegebenenfalls linear oder verzweigt vorliegen sowie einfach oder mehrfach mit Halogenen, N-, O-, P-, S-atomhaltigen Resten substituiert sein können, (C₃-C₇)-Cycloalkyl, welche gesättigt oder ungesättigt sowie einfach oder mehrfach mit linear oder verzweigten (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein oder im Ring Heteroatome wie N, O, P, S enthalten können, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die eben genannten Cyclen gegebenenfalls einfach oder mehrfach mit linear oder verzweigten (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein können, N-verknüpfter Aminosäure- oder Peptidrest,
R² bedeuten kann H, (C₁-C₈)-Alkyl, (C₂-C₈)-Alkenyl, (C₂-C₈)-Alkinyl, (C₂-C₈)-Alkoxyalkyl, welche gegebenenfalls linear oder verzweigt vorliegen sowie einfach oder mehrfach mit Halogenen, N-, O-, P-, S-atomhaltigen Resten substituiert sein können, (C₃-C₇)-Cycloalkyl, welche gesättigt oder ungesättigt sowie einfach oder mehrfach mit linear oder verzweigten (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein und/oder im Ring Heteroatome wie N, O, P, S enthalten können, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die eben genannten Cyclen gegebenenfalls einfach oder mehrfach mit linear oder verzweigten (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein können,
R³ bedeuten kann H, ClCO, (C₁-C₈)-Acyl, welcher gegebenenfalls linear oder verzweigt vorliegen kann, ein C-verknüpfter Aminosäure- oder Peptidrest oder eine gängige Peptidschutzgruppe wie z. B. Formyl, Carbamoyl, Benzyloxycarbonyl, tert.-Butyloxycarbonyl, Allyloxycarbonyl, Trifluoracetyl.

Weiterhin richtet sich die Erfindung auf neue Zwischenprodukte der allgemeinen Formel V, IV und II sowie deren Salze 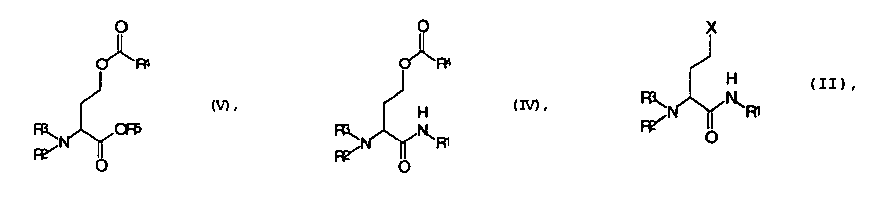 worin R¹, die oben angegebene Bedeutung besitzen, R² für H, R³ für Formel II die oben angegebene Bedeutung annimmt und in Formel IV und V für Benzyloxycarbonyl oder tert.-Butyloxycarbonyl oder Trifluoracetyl steht und R⁴ für Methyl sowie R⁵ für H steht,oder R⁵ und R³ über eine C=O-Gruppe zu einem Ring verbunden sind und deren Verwendungen.

Die durch das erfindungsgemäße Verfahren herstellbaren Verbindungen und die neuen Zwischenprodukte sind wertvolle Intermediate zur Produktion von bioaktiven Wirkstoffen. So werden 3-Amino-2-oxo-pyrrolidine bevorzugt als Baustein für Peptidmimetika eingesetzt, die als Pharmazeutika verwendet werden. In der WO 94/22820 sind beispielsweise am Phenylring substituierte 3-Amino-1-phenyl-2-oxo-pyrroldine als Zwischenprodukte für Thrombozyten Aggregationsinhibitoren beschrieben. Weitere, diese γ-Lactame enthaltende bioaktive Verbindungen sind von Kottirsch et al. (Bioorg. Med. Chem. Lett. 1993, 3, 1675) untersucht worden. In anderen Beispielen werden diese in hochpotenten Neurokinin NK-2 Rezeptor Antagonisten nach Deal et al. (J. Med. Chem. 1992, 35, 4195) eingesetzt.

Die Mehrzahl der bisher angewandten Verfahren zur Herstellung von substituierten 3-Amino-2-oxo-pyrrolidinen besteht darin, die entsprechenden offenkettigen Methioninverbindungen zunächst in deren Sulfoniumsalze zu überführen und diese mit starken Basen in einem geeigneten Lösungsmittel zu cyclisieren. Von Friedinger et. al (J. Org. Chem. 1982, 47, 104-109) wurde hierfür Methyliodid und Natriumhydrid, was im großtechnischen Prozeß schlecht handhabbaren ist, eingesetzt. In der US 5,484,946 werden für die Alkylierung anstelle des leichtflüchtigen Methyliodids Trimethylsulfonium- bzw. Trimethylsulfoxoniumsalze eingesetzt. Die Cyclisierung wird dann mit Kaliumcarbonat durchgeführt.

Das Hauptproblem dieser Prozedere, nämlich die zwangsläufige Freisetzung des extrem geruchsintensiven Dimethylsulfids aus der Methioninvorstufe, kann jedoch auch mit dem eben genannten Verfahren nicht vermieden werden. Nachteilig erscheint darüber hinaus der erforderliche Einsatz von teuren aprotisch polaren Lösungsmitteln wie z. B. DMSO bei der Cyclisierung mit Kaliumcarbonat.

In der WO 94/22820 wird zwar ein Verfahren erwähnt, bei dem racemische Homoserinderivate, die ausgehend von Butyrolacton hergestellt wurden, mittels Triphenylphosphin und Azodicarbonsäurediestern zu Pyrrolidonen cyclisiert werden. Diese Reagenzien sind jedoch für den Einsatz in einem technischen Verfahren wenig geeignet, da sie relativ teuer sind. Desweiteren liefert diese Variante bei der Cyclisierung eine Reihe von Nebenprodukten, deren Abtrennung vom gewünschten Derivat schwierig und damit zeit- und kostenintensiv ist (K. Nakajima et al. Peptide Chemistry 1983, 77-80).

Obwohl L-Homoserin eine natürlich vorkommende Aminosäure ist, sind bisher nur relativ wenig Synthesen von Homoserinamiden, z. B. Peptiden, bekannt, die von Homoserin ausgehen. Die Gründe dafür sind, daß Homoserin sowie die entsprechenden N-Acylverbindungen unter sauren Bedingungen sehr leicht die entsprechenden Lactone bilden (J. P. Greenstein, M. Winitz, "Chemistry of the Amino Acids", Wiley, New York 1961, Bd. 3, S. 2612). Das gleiche geschieht auch, wenn die Carboxygruppe aktiviert wird, wie es für die Herstellung von Homoserinamiden erforderlich ist.

Zwar können auch N-Acylhomoserinlactone mit Alkylaminen sowie Aminosäureestern bzw. Aminosäuresalzen zu den entsprechenden Amiden umgesetzt werden (Sheradsky et al., J. Org. Chem. 1961, 26, 2710), hierfür sind jedoch entweder sehr lange Reaktionszeiten oder höhere Temperaturen erforderlich. Für die Herstellung von komplexen eventuell optisch aktiven Verbindungen hat diese Methode daher keine Anwendung gefunden.

Zur Herstellung von Homoserinamiden werden Homoserinderivate eingesetzt, bei denen die Hydroxyfunktion durch eine geeignete Gruppe geschützt ist. Dies wurde bisher entweder durch eine Tritylgruppe (Barlos et al., J. Chem. Soc., Chem. Commun. 1986, 1259), durch Mono- bzw. Dimethoxytritylgruppen (Beltran et al., Lett. Pept. Sci. 1997, 4, 147), tert.-Butyldimethylsilylgruppe (WO 97/46248) oder Benzylgruppen (Cornille et al., J. Am. Chem. Soc. 1995, 117, 909) erreicht. Nachteilig an diesen Schutzgruppen ist, daß diese entweder teure Chemikalien erfordern oder aber nur umständlich einzuführen sind.

Einfache und kostengünstige O-Schutzgruppen könnten O-Acylverbindungen sein. Hierbei tritt jedoch das Problem auf, daß diese unter basischen Bedingungen sehr schnell einen O -> N-Acylshift erleiden unter Bildung der entsprechenden N-Acylhomoserine. Weiterhin ist zur Herstellung von O-Acetylhomoserin bisher nur die Umsetzung von Homoserin mit Acylanhydriden in Perchlorsäure beschrieben, was wegen der Explosionsneigung von Perchloraten für größere Ansätze sehr nachteilig und nicht geeignet erscheint. Die Ausbeuten liegen lediglich bei max. 51% (Nagani et al., J. Biol. Chem. 1967, 242, 3884). <5a>

Aufgabe der vorliegenden Erfindung ist deshalb die Angabe eines weiteren Verfahrens zur Herstellung von γ-Lactamen der allgemeinen Formel I, welches vorteilhaft in einem technischen Maßstab durchführbar ist, d. h. möglichst den Einsatz von kritischen Substanzen sowie teuren Reagenzien vermeidet. Unter kritischen Substanzen werden im Sinne der Erfindung solche Verbindungen verstanden, die im großen Aus K. UNDHEIM, G. A. ULSAKER: "N-Quaternary Compounds" ACTA CHEM: SCAND., Bd. 27, Nr. 3, 1973, Seiten 1059-1066 , ist die intramolekulare Kondensation von Verbindungen der Formel II, die anstelle des Substituenten X ein Chloratom tragen, zu Verbindungen der Formel I mittels methanolischer Natriummethylatlösung bekannt. Natriummethylat ist eine sehr starke Base, welche bei empfindlichen Einsatzstoffen zu vermehrter Nebenproduktbildung Anlass geben kann.

Maßstab eingesetzt zu besonderen Risiken hinsichtlich der Umweltverschmutzung oder des Arbeitsschutzes Anlaß geben.

Aufgabe der vorliegenden Erfindung ist es auch, ein Verfahren anzugeben, welches die Herstellung der γ-Lactame der allgemeinen Formel I ohne den Anfall des übelriechenden Dimethylsufids gestattet.

Weiterhin ist Aufgabe der Erfindung, neue vorteilhaft für die Synthese der γ-Lactame der allgemeinen Formel I einsetzbare Zwischenprodukte anzugeben und eine vorteilhafte Verwendung dieser Zwischenprodukte zu nennen.

Diese und weitere nicht näher erläuterte Aufgaben, welche sich jedoch ohne weiteres in naheliegender Weise aus dem Stand der Technik ergeben, sind Gegenstand eines Verfahrens mit den Merkmalen des kennzeichnenden Teils des Anspruchs 1. Vorteilhafte Ausgestaltungen des erfindungsgemäßen Verfahrens werden in den von Anspruch 1 abhängigen Unteransprüchen beschrieben. Ansprüche 26 bis 34 lösen die Aufgabe zur Angabe von neuen Zwischenprodukten. Unter Anspruch 35 wird eine vorteilhafte Verwendung der Zwischenprodukte beschreiben.

Dadurch, daß man eine Verbindung der allgemeinen Formel I und deren Salze 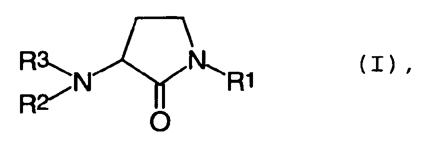 worin
R¹ bedeuten kann H, (C₁-C₈)-Alkyl, (C₂-C₈)-Alkenyl, (C₂-C₈)-Alkinyl, (C₂-C₈)-Alkoxyalkyl, (C₁-C₈)-Acyl, welche gegebenenfalls linear oder verzweigt vorliegen sowie einfach oder mehrfach mit Halogenen, N-, O-, P-, S-atomhaltigen Resten substituiert sein können, (C₃-C₇)-Cycloalkyl, welche gesättigt oder ungesättigt sowie einfach oder mehrfach mit linear oder verzweigten (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein und/oder im Ring Heteroatome wie N, O, P, S enthalten können, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die eben genannten Cyclen gegebenenfalls einfach oder mehrfach mit linear oder verzweigten (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein können, N-verknüpfter Aminosäure- oder Peptidrest,
R² bedeuten kann H, (C₁-C₈)-Alkyl, (C₂-C₈)-Alkenyl, (C₂-C₈)-Alkinyl, (C₂-C₈)-Alkoxyalkyl, welche gegebenenfalls linear oder verzweigt vorliegen sowie einfach oder mehrfach mit Halogenen, N-, O-, P-, S-atomhaltigen Resten substituiert sein können, (C₃-C₇)-Cycloalkyl, welche gesättigt oder ungesättigt sowie einfach oder mehrfach mit linear oder verzweigten (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein und/oder im Ring Heteroatome wie N, O, P, S enthalten können, Aryl, wie Phenyl oder Naphthyl, Aralkyl, wie Benzyl oder Phenethyl, Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl,
wobei die eben genannten Cyclen gegebenenfalls einfach oder mehrfach mit linear oder verzweigten (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein können,
R³ bedeuten kann H, ClCO, (C₁-C₈)-Acyl, welcher gegebenenfalls linear oder verzweigt vorliegen kann, ein C-verknüpfter Aminosäure- oder Peptidrest oder eine gängige Peptidschutzgruppe wie z. B. Formyl, Carbamoyl, Benzyloxycarbonyl, tert.-Butyloxycarbonyl, Allyloxycarbonyl, Trifluoracetyl,
durch Cyclisierung von Derivaten der allgemeinen Formel II 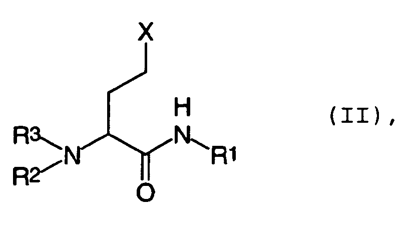 in der R¹, R², R³ die oben angegebene Bedeutung besitzen und X Sulfonsäureester darstellt, synthetisiert, gelangt man in sehr einfacher und dennoch vorteilhafter Weise in guten Ausbeuten zu den gewünschten Verbindungen der allgemeinen Formel I, wobei die für diese Synthese notwendigen relativ preisgünstigen Einsatzstoffe, die einfache Reaktionsführung, die guten Ausbeuten und der Verzicht auf gefährliche oder übelriechende Reagenzien dieses erfindungsgemäße Verfahren für einen großtechnischen Prozeß prädestinieren.

Als Sulfonsäurerest X werden im Rahmen des erfindungsgemäßen Verfahrens alle sich von einer Sulfonsäure der Struktur HOSO₃R' ableitenden Reste verstanden, wobei R' in diesem Zusammenhang einen linearen oder verzweigten (C₁-C₈)-Alkylrest oder einen Arylrest, welcher gegebenenfalls mit einem oder mehreren (C₁-C₈)-Alkylresten substituiert sein kann, darstellt. Die genannten Reste können gegebenenfalls mit einem oder mehreren Elementen der Gruppe Halogen, bevorzugt Cl oder F substituiert sein.

Vorteilhafterweise kann die eben beschriebene Cyclisierung dabei unter basischen Bedingungen erfolgen, wobei als Base bevorzugt ein Alkalihydroxid eingesetzt werden kann. Besonders bevorzugt ist der Einsatz von wäßriger Alkalihydroxidlösung, und ganz besonders bevorzugt kann Natriumhydroxidlösung zur Cyclisierung verwendet werden. Vorteilhaft für die Cyclisierung einzusetzende Verbindungen der allgemeinen Formel II besitzen als Abgangsgruppe X einen Sulfonsäureester und besonders bevorzugt kann die sogenannte Mesyl(OSO₃Me)-Gruppe eingesetzt werden.

Ganz besonders bevorzugt ist dieses Verfahren auf Verbindungen der allgemeinen Formel II anzuwenden, bei denen R¹ einen p-Cyanophenyl- oder p-Carbamoylphenylrest darstellt, während R² vorteilhafterweise H und R³ = Benzyloxycarbonyl ist.

Die Cyclisierung kann bei Temperaturen von -20°C bis 100 °C betrieben werden. Bevorzugt werden Temperaturen von 0°C bis 50 °C, ganz besonders bevorzugt Temperaturen von 10°C bis 30 °C, bei der Cyclisierung eingestellt.

Die Synthese der Verbindungen der allgemeinen Formel I kann bevorzugt und äußerst einfach ausgehend von Verbindungen der allgemeinen Formel II durchgeführt werden, wenn II ohne vorherige Isolierung zu I umgesetzt wird.

Die Verbindungen der allgemeinen Formel II lassen sich vorteilhafterweise aus Derivaten der allgemeinen Formel III 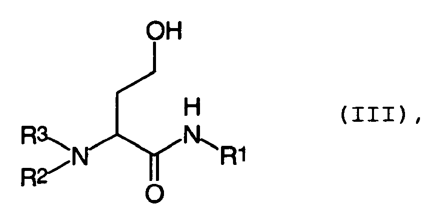 worin R¹, R², R³ die oben angegebene Bedeutung besitzen, durch Sulfonsäureesterbildung herstellen. Bevorzugt werden die Verbindungen der allgemeinen Formel III vor der Weiterverarbeitung zu Verbindungen der Formel II nicht isoliert.

Die Verbindungen der Formel III wiederum werden ganz besonders bevorzugt aus Verbindungen der allgemeinen Formel IV 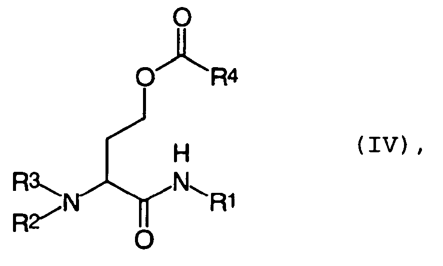 worin R¹, R², R³ die oben angegebene Bedeutung besitzen und
R⁴ für (C₁-C₈)-Alkyl, (C₂-C₈)-Alkenyl, (C₁-C₈)-Alkoxy, (C₂-C₈)-Alkenyloxy, welche gegebenenfalls linear oder verzweigt vorliegen und ggf. mit einem oder mehreren Halogenatomen substituiert sind,
Aryl, wie Phenyl oder Naphthyl,
Aralkyl, wie Benzyl oder Phenethyl,
Arylalkyloxy, wie Benzyloxy, hergestellt.

Im Prinzip können für die eben genannte Transformation alle dem Fachmann bekannten Verfahren zur Verseifung benutzt werden. Vorteilhafterweise können Verbindungen der allgemeinen Formel IV aber durch Aminolyse in Verbindungen der allgemeinen Formel III überführt werden. Besonders bevorzugt ist der Einsatz von Ammoniak als Amin in diesem Zusammenhang, ganz besonders bevorzugt kann Ammoniak als seine wäßrige Lösung eingesetzt werden.

Die Verbindung der allgemeinen Formel IV wiederum können vorteilhafterweise aus Verbindungen der allgemeinen Formel V 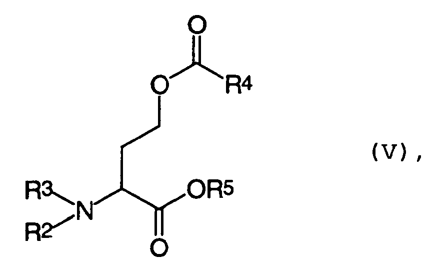 in der R², R³, R⁴ die oben angegebene Bedeutung besitzen und R⁵ für H steht oder worin R³ und R⁵ über eine C=O-Gruppe zu einem Ring verbunden sind, hergestellt werden. Dabei können Verbindungen der allgemeinen Formel V in denen R⁵ für H steht vor der Umsetzung zu Verbindungen der allgemeinen Formel IV bevorzugt durch Säurechloride aktiviert werden (analog Houben-Weyl, Band 15, Teil 2, S. 169 ff). Verbindungen der allgemeinen Formel V, in denen R³ und R⁵ über eine C=O-Gruppe zusammen zu einem Ring verbunden sind, kann man vorteilhafterweise in Verbindungen der allgemeinen Formel IV überführen, in denen R³ für H steht.

Erfindungsgemäß lassen sich die Verbindungen der allgemeinen Formel V vorteilhaft aus den Säureadditionssalzen der allgemeinen Formel VI 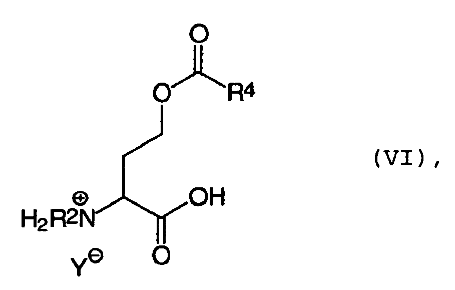 in der R², R⁴ die oben angegebene Bedeutung hat und Y^{⊖} die korrespondierende Base einer anorganischen Säure ist, durch Umsetzen mit einem sich von R³ ableitenden Acylierungsreagenz herstellen. Unter einer anorganischen Säure wird im Rahmen der Erfindung eine Säure verstanden, deren pKa-Wert 2.5 unterschreitet, wie z.B. HCl, HBr, H₂SO₄, H₃PO₄. Als Acylierungsreagenz kann bevorzugt Phosgen eingesetzt werden. In diesem Fall erhält man Verbindungen der allgemeinen Formel V, in denen R³ und R⁵ zusammen über eine C=O-Gruppe zu einem Ring verbunden sind. Alternativ lassen sich Verbindungen der allgemeinen Formel VI durch Umsetzen mit Acylierungsreagenzien der Gruppe der organischen Anhydride, wie z.B. Di-tert.-butylpyrocarbonat, der aktivierten Ester, wie z.B. Trifluoressigsäureester, Essigsäurehydroxysuccinimidester, oder der Halokohlensäureester, wie z.B. Benzyloxycarbonylchlorid, Allyloxycarbonylchlorid oder tert.-Butyloxycarbonylfluorid, erhalten. Die wie eben beschriebene Acylierung kann bevorzugt in wäßriger Lösung bei einem pH-Wert 4-9 erfolgen, besonders bevorzugt ist die Einhaltung eines pH-Intervalls von 6.5-7.5 bei dieser Reaktion. Die Acylierung zu Verbindungen der allgemeinen Formel V mit R⁵ gleich H kann allerdings auch in organischen Lösungsmitteln in Gegenwart einer tertiären Base durchgeführt werden. Als tertiäre Base kann vorteilhafterweise Triethylamin dienen.

Das Säureadditionssalz der allgemeinen Formel VI wiederum kann bevorzugt aus Verbindungen der allgemeinen Formel VII 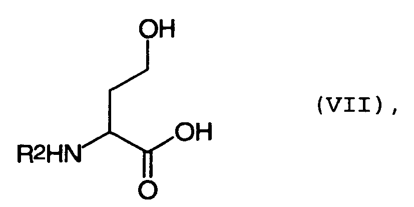 in der R² die oben angegebene Bedeutung hat,
hergestellt werden. Dies kann in einem Gemisch aus einer Carbonsäure R⁴COOH und dem Carbonsäurechlorid oder -bromid R⁴COCl bzw. R⁴COBr, wobei R⁴ die oben angegebene Bedeutung hat, erfolgen. Es ist vorteilhaft für diese Umsetzung, zuerst die entsprechende Carbonsäure R⁴COOH und das entsprechende Carbonsäurehalogenid R⁴COHal miteinander zu mischen und anschließend die Verbindung der allgemeinen Formel VII zu dieser Mischung hinzuzufügen. Bei dem eben genannten Verfahren arbeitet man vorteilhafterweise bei einer Temperatur zwischen -20°C und 50 °C, bevorzugt zwischen -10°C und 20 °C und ganz besonders bevorzugt zwischen -5°C und 10 °C.

Eine weitere bevorzugte Variante zur Herstellung der Verbindung der allgemeinen Formel V besteht darin, daß Verbindungen der allgemeinen Formel VIII 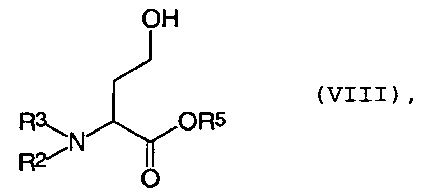 in der die Reste R², R³ die oben angegebene Bedeutung innehaben können und R⁵ für H steht, mit dem Reagenz R⁴COZ, worin R⁴ die oben angegebene Bedeutung besitzt und Z einen aktivierenden Rest darstellt, umsetzt. Der aktivierende Rest Z kann prinzipiell alle dem Fachmann für diesen Zweck geläufige Ausgestaltungen wie z.B. Hydroxysuccinimid, Hydroxybenzotriazol, R⁴CO₂, Halogen annehmen (The Chemical Synthesis of Peptides, J. Jones, Oxford Press 1991,S 42 ff). Bevorzugt werden zur Acylierung der OH-Funktion der Formel VIII Verbindungen genommen, bei denen der aktivierende Rest Z einem Halogen entspricht. In diesem Fall wird die Reaktion ganz besonders bevorzugt in der dem Carbonsäurehalogenid korrespondierenden Carbonsäure als Lösungsmittel ausgeführt. Als Halogenide sind die Chloride bzw. Bromide der Carbonsäure äußerst bevorzugt. Ganz besonders bevorzugt ist dabei die Ausführungsform, in der die bei der Acylierung entstehende anorganische Säure durch Zugabe des Natriumsalzes der korrespondierenden als Lösungsmittel eingesetzten Carbonsäure abgepuffert wird.

In einem weiteren Aspekt der vorliegenden Erfindung werden vorteilhafte neue Zwischenprodukte der allgemeinen Formel V, IV und II sowie deren Salze 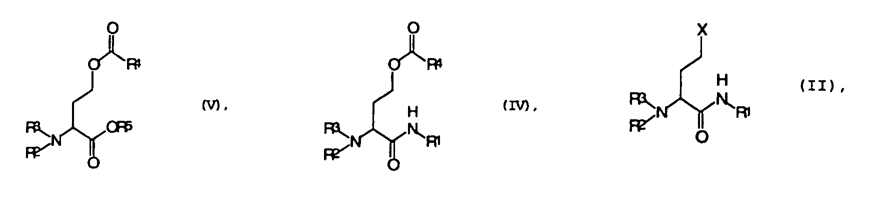 in denen R¹ die oben angegebene Bedeutung besitzt, R² für Wasserstoff, R³ für Formel II die oben angegebene Bedeutung annimmt und in Formel IV und V für Benzyloxycarbonyl oder tert.-Butyloxycarbonyl oder Trifluoracetyl, R⁴ einen Methylrest und R⁵ einen Wasserstoffrest darstellt oder wo R³ und R⁵ zusammen über eine C=O-Gruppe zu einem Ring verbunden sind. X ist bevorzugt ein Sulfonsäureester, ganz besonders bevorzugt eine OSO₃Me-Gruppe. R¹ ist ganz besonders bevorzugt p-Cyanophenyl oder p-Carbamoylphenyl.

Die vorteilhaften Verbindungen der allgemeinen Formel V, IV und II werden erfindungsgemäß zur Herstellung von Intermediaten von bioaktiven Wirkstoffen eingesetzt.

Die vorliegende Erfindung ist im nachfolgenden Schema nochmals verdeutlicht:

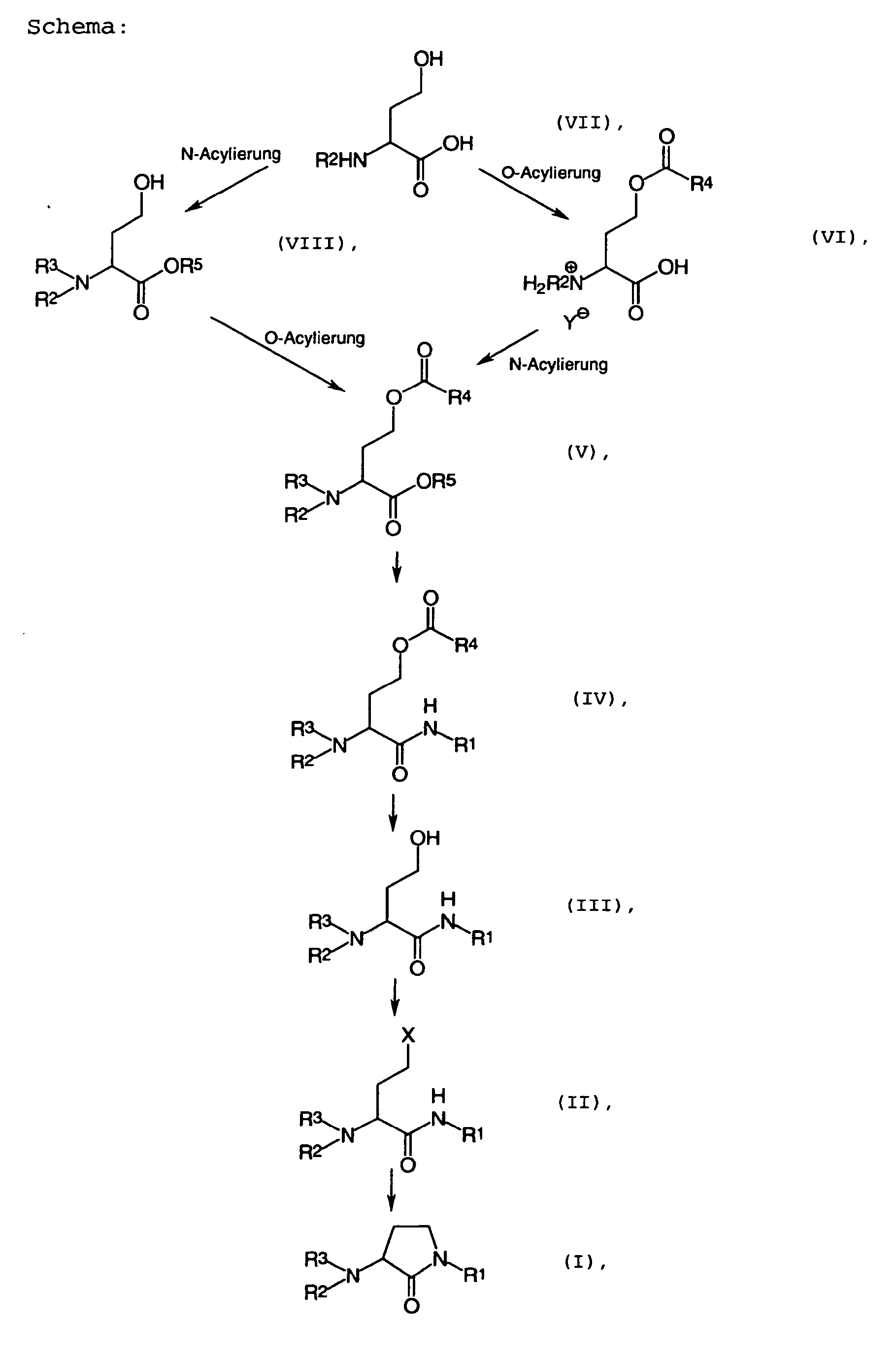

Die Cyclisierung von II nach I ist eine intramolekulare nucleophile Substitution. Prinzipiell sind alle dem Fachmann geläufigen Varianten für diese Reaktion heranzuziehen. Allerdings ist bei dieser Reaktion mit einer Reihe von Nebenprodukten, wie z.B. der Eliminierung oder Substitution der Abgangsgruppe, zu rechnen. Darum war es sehr überraschend, daß für die Cyclisierung mit der Auswahl der Abgangsgruppen X Bedingungen gefunden werden konnten, die den Einsatz einer relativ starken Base - hier Natriumhydroxyd - in Gegenwart eines polaren protischen, Kationen stabilisierenden Lösungsmittel - hier Wasser - erlauben und trotzdem eine weitestgehend selektiv verlaufende Reaktionsführung gestatten. Diese sehr preiswerten und gut für einen großtechnischen Prozess einsetzbaren unkritischen Reagenzien sind anderen Bedingungen aus dem Stand der Technik für die intramolekulare Substitution klar vorzuziehen (keine Dimethylsulfidbildung, kein Natriumhydrideinsatz, keine teuren Reagenzien und Lösungsmittel). Weiterhin vorteilhaft bei diesen bevorzugten Umsetzungsbedingungen ist, daß die optische Aktivität von enantiomer angereicherten Ausgangsverbindungen unter den erfindungsgemäßen Bedingungen nahezu vollständig erhalten bleibt.

Bevorzugt kommen in der eben dargestellten Reaktion Derivate von II zum Einsatz, die als Rest R¹ die p-Cyanophenyl- oder p-Carbamoylphenylgruppe, für R² H und für R³ einen Benzyloxycarbonyl-Rest beinhalten. Diese führen zu vorteilhaften Verbindungen für die Herstellung von Pharmazeutika wie z.B. in der WO94/22820 beschrieben.

Die Herstellung der Verbindungen der Formel II aus III kann analog literaturbekannter Verfahren erfolgen. Bevorzugt wird jedoch aus III und Sulfonsäurechlorid ein Sulfonsäureester gebildet, der vorteilhafterweise ohne Zwischenisolierung wie oben angegeben weiter zu I umgesetzt werden kann. Ganz besonders bevorzugt wird die Mesyl (OSO₃Me)-Gruppe, die mit Mesylchlorid und Triethylamin als Base eingeführt werden kann, eingesetzt. Es ist erstaunlich, daß unter den bevorzugten erfindungsgemäßen Reaktionsbedingungen nicht die Hydrolyse des Sulfonsäureesters II sondern die Cyclisierung die Hauptreaktion ist. Dies war nicht vorhersehbar. Besonders vorteilhaft ist weiterhin, daß die Verbindungen der allgemeinen Formel II vor ihrer Umsetzung zu I nicht isoliert werden müssen. Sie werden gebildet und anschließend bevorzugt in situ umgesetzt.

Die Herstellung der Verbindungen der allgemeinen Formel III aus den Verbindungen der allgemeinen Formel IV kann durch saure oder basische Hydrolyse der O-Acylgruppe erfolgen. Unter diesen Bedingungen kann jedoch eine teilweise Hydrolyse beispielsweise der ggf. vorhandenen N-Acylgruppe und der Amidbindung eintreten. Unter der bevorzugten Hydrolysevariante wird die Abspaltung der O-Acylgruppe daher durch Aminolyse durchgeführt. Besonders bevorzugt wird dafür Ammoniak, ganz besonders bevorzugt seine wäßrige Lösung eingesetzt. Unter diesen Bedingungen wird selektiv die O-Acylgruppe abgespalten.

Erfindungsgemäß ist es ebenfalls nicht erforderlich, die Verbindungen der allgemeinen Formel III zu isolieren. Diese können auch direkt zu Verbindungen der allgemeinen Formel II umgesetzt werden. Es ist deshalb für das vorliegende Verfahren ganz außerordentlich vorteilhaft eine Verfahrensvariante zu wählen, bei der ausgehend von Derivaten der allgemeinen Formel IV bis zum Endprodukt der allgemeinen Formel I keine Zwischenisolierung einer Zwischenstufe erfolgt. Trotzdem erhält man die Derivate der allgemeinen Formel I in guten bis sehr guten Ausbeuten und mit einer sehr guten Reinheit behaftet. Dies war überraschend und keinesfalls vorhersehbar, dabei für ein großtechnisches Verfahren aber umso vorteilhafter, da die sehr kostenintensive Handhabung von isolierten Zwischenprodukten auf ein Minimum reduziert werden kann.

Als Lösungsmittel für die Umwandlung III->II können alle organischen Lösungsmittel verwendet werden, die unter den gegebenen Bedingungen inert reagieren. Besonders bevorzugt sind Ether, wie z.B. Methyl-tert.-butylether, Tetrahydrofuran, Dimethoxyethan, Dioxan, Kohlenwasserstoffe, wie z.B. Hexan, Cyclohexan, Toluol, Ketone, wie z.B. Aceton, Diethylketon, Methylisobutylketon und Carbonsäurealkylester, wie z.B. Ethylacetat, Isopropylacetat, n-Butylacetat. Ganz besonders bevorzugt sind organische Lösungsmittel, die mit Wasser ein niedrigsiedendes Azeotrop bilden, wie z.B. Toluol, Methylisobutylketon oder n-Butylacetat.

Verbindungen der Formel IV wiederum können bevorzugt aus Derivaten V gewonnen werden. In dem Fall, in dem die Reste R³ und R⁵ über eine C=O-Gruppe miteinander zu einem Ring verbunden sind, also ein N-Carbonsäureanhydrid des betrachteten Derivats vorliegt, muß die Carboxyfunktion des Homoserinderivats für die Kopplung nicht mehr aktiviert werden. Man setzt dann einfach mit dem Amin der Wahl um und erhält Verbindungen der Formel IV mit R³ gleich H. Diese können nach Literaturverfahren durch Umsetzung mit einem sich von R³ ableitenden Acylierungsreagenz zu den entsprechenden N-Acylverbindungen umgesetzt werden.

Im Falle von R⁵ = H wird die Carboxyfunktion vor der Kopplung mit dem Amin aktiviert. Dies erfolgt nach dem Fachmann bekannten Methoden der Peptidchemie (Houben-Weyl, Band 15, 2. Teil), wie z.B. über gemischte Anhydride, Aktivester etc. Bevorzugt ist jedoch die Umsetzung mit einem Säurechlorid wie z.B. Pivaloylchlorid zum gemischten Anhydrid.

Die erfindungsgemäßen Verbindungen der allgemeinen Formel V lassen sich auf zwei unterschiedlichen Wegen aus Homoserinderivaten wie VII erhalten. Entweder wird VII erst am Sauerstoff zu VI acyliert und anschließend am Stickstoff durch N-Acylierung zu V weiterverarbeitet, oder Verbindungen der allgemeinen Formel VIII, welche durch N-Acylierung aus VII erhältlich sind, werden durch O-Acylierung zu V umgesetzt.

Die N-Acylierung zum Homoserinderivat VIII kann analog zu der von anderen Aminosäuren erfolgen (Houben-Weyl, Band 15, Teil 1 und Teil 2). Als Acylierungsreagenzien können prinzipiell alle sich von R³ ableitenden Acylierungsreagenzien wie Aminoschutzreagenzien, C-terminal aktivierte Aminosäuren oder Peptide fungieren.

Bei der Isolierung der N-Acylhomoserine der Formel VIII bestand jedoch das Problem, daß diese als freie Säure sehr unbeständig sind und leicht zu den entsprechenden Lactonen cyclisieren. Diese Cyclisierung wird durch Säuren katalysiert und tritt darüberhinaus immer dann auf, wenn die Säurefunktion aktiviert wird. Dies erfolgt auch bei der Umsetzung mit dem aktivierten Säurederivat R⁴COZ unter den für Acylierungen sonst üblichen basischen Bedingungen. Wird die O-Acylierung allerdings in dem zur O-Acylierung benutzten Reagenz R⁴COZ korrespondierenden Carbonsäure R⁴COOH als Lösungsmittel durchgeführt, so erhält man fast ausschließlich die gewünschten Derivate V. Besonders bevorzugt ist der Einsatz von Cl für Z. Optional und vorteilhaft benutzt man eine Base zur Abpufferung der bei dieser Reaktion entstehenden Säure. Vorteilhafterweise setzt man das Natriumsalz der als Lösungsmittel eingesetzten Carbonsäure als Base zu. Das auch nach diesem Verfahren jedoch in untergeordnetem Maße entstehende Nebenprodukt, das Lacton, kann einfach durch Extraktion vom Produktgemisch abgetrennt werden und steht nach Ringöffnung erneut für die Reaktion zur Verfügung.

Bei der zweiten Variante erfolgt die O-Acylierung zu Derivaten VI erfindungsgemäß in einem Medium, welches die N-Acylierung verhindern hilft, da dies die schnellere Reaktion wäre. Bevorzugt wird die Umsetzung deshalb in einer Carbonsäure wie R⁴COOH, die vorher mit dem entsprechenden korrespondierenden Carbonsäurehalogenid, bevorzugt Carbonsäurechlorid, versetzt wurde, durchgeführt. Bei der folgenden Zugabe des Homoserinderivats VII kommt es durch die im Lösungsmittel sich bildende starke anorganische Säure durch Protonierung zu einem Schutz der Aminofunktion von VII und es erfolgt bevorzugt eine Acylierung der Hydroxyfunktion. Als Produkt wird somit das Säureadditionssalz des hydroxygeschützten Homoserins VI erhalten. Ein Einsatz der gefährlichen Perchlorsäure, wie aus dem Stand der Technik bekannt, wird somit umgangen. Weiterhin sind nach dem erfindungsgemäßen Verfahren die Ausbeuten deutlich besser.

Die Weiterreaktion von Derivaten VI zu V kann prinzipiell nach den Fachmann bekannten Verfahren mit einem sich von R³ ableitenden Acylierungsreagenz wie Aminoschutzreagenzien, C-terminal aktivierte Aminosäuren, Peptide oder mit Phosgen erfolgen. Hierbei bestand jedoch das Problem, daß die O-Acylhomoserine VI unter basischen Bedingungen schnell einen O->N-Acylshift eingehen. Nach dem erfindungsgemäßen Verfahren kann dies vermieden werden, indem man, falls man die Acylierung in wäßrigem Milieu durchführt, den pH-Bereich zwischen 3 und 9, bevorzugt 6 und 7.5, hält. Eine weitere Möglichkeit wird in der Umsetzung der O-Acylverbindungen VI in wasserfreien Lösungsmitteln und Triethylamin als Base gefunden. Für die Umsetzung mit Phosgen kann das Säureadditionssalz VI ohne Zusatz einer Base eingesetzt werden.

Als linear oder verzweigte (C₁-C₈)-Alkylreste sind anzusehen Methyl, Ethyl, n-Propyl, Isopropyl, n-Butyl, Isobutyl, sec-Butyl, tert.-Butyl, Pentyl, Hexyl, Heptyl oder Octyl samt aller ihrer Bindungsisomeren. Der linear oder verzweigte (C₂-C₈)-Alkenylrest umfaßt alle Substituenten, welche eben beim (C₁-C₈)-Alkylrest aufgezählt wurden mit Ausnahme vom Methylrest, wobei in diesen Resten mindestens eine Doppelbindung vorhanden ist. Der Umfang von (C₂-C₈)-Alkinyl entspricht dem von (C₂-C₈)-Alkenyl, wobei hier allerdings mindestens eine Dreifachbindung vorliegen muß. Der Rest (C₁-C₈)-Alkoxy entspricht dem Rest (C₁-C₈)-Alkyl mit der Maßgabe, daß dieser über ein Sauerstoffatom an den Cyclus gebunden ist. Als (C₂-C₈)-Alkoxyalkyl sind Reste gemeint, bei denen die Alkylkette durch mindestens eine Sauerstoffunktion unterbrochen ist, wobei nicht zwei Sauerstoffe miteinander verbunden sein können. Die Anzahl der Kohlenstoffatome gibt die Gesamtzahl der im Rest enthaltenen Kohlenstoffatome an. Als (C₂-C₈)-Alkenyloxy versteht man Reste wie (C₂-C₈)-Alkoxy, welche jedoch mindestens eine C-C-Doppelbindung aufweisen.
N-, O-, P-, S-atomhaltige Reste sind insbesondere Alkyl, Alkenyl, Alkinyl-Reste der oben genannten Art, welche eines oder mehreren dieser Heteroatome in ihrer Kette aufweisen bzw. welche über eines dieser Heteroatome an das Molekül gebunden sind. Unter (C₃-C₇)-Cycloalkyl versteht man Cyclopropyl, Cyclobutyl, Cyclopentyl, Cyclohexyl bzw. Cycloheptylreste.

Als Halogene kommen Fluor, Chlor, Brom und Iod in Frage.

Unter N-verknüpftem Aminosäure- oder Peptidrest wird eine Verbindung angesehen, bei der das in Frage stehende Molekül über sein N-Atom an das α-C-Atom einer sich von einer Aminosäure ableitenden Carbonsäure, welche Bestandteil eines Peptidrestes sein kann, gebunden ist.

Unter C-verknüpftem Aminosäure- oder Peptidrest wird eine Verbindung angesehen, bei der das in Frage stehende Molekül über sein N-Atom an das C-Atom der Carboxylgruppe einer Aminosäure, welche Bestandteil eines Peptids sein kann, gebunden ist.

Unter (C₁-C₈)-Acyl versteht man einen Alkylrest mit einem bis acht C-Atomen, der linear oder verzweigt vorliegen kann, also ebenfalls die gesamten möglichen Bindungsisomere mitumfaßt, welcher über eine C=O-Funktion an das Molekül gebunden ist.

Verbindungen mit einem stereogenen Zentrum bedeuten im Rahmen der Erfindung Racemate sowie die jeweiligen enantiomeren Antipoden dieser Strukturen. Für das erfindungsgemäße Verfahren bevorzugt ist allerdings der Einsatz enantiomerenangereicherter Homoserinderivate der allgemeinen Formel VII. So erhält man bei Einsatz der enantiomerenangereicherten Edukte (D oder L) unter nahezu vollständiger Stereokonservation die enantiomerenangereicherten Produkte der allgemeinen Formel V und I (D oder L).

Unter Salzen versteht man ionische Additionsverbindungen aus starken Säuren wie HCl, HBr, H₂SO₄, H₃PO₄, CF₃COOH, p-Toluolsulfonsäure, Methansulfonsäure und dem betrachteten Molekül.

Die folgenden Beispiele sollen die Erfindung erläutern.

### Beispiele:

### O-Acetyl-L-homoserin Hydrochlorid (VI)

Zu einer Lösung von 500 ml Essigsäure und 500 ml Acetylchlorid werden unter Kühlung bei < 30 °C portionsweise 72 g (0.60 mol) L-Homoserin gegeben. Nach 2 h Nachrühren kann mit DC keine Homoserin nachgewiesen werden. Der Niederschlag wird abgesaugt und mit Essigsäure nachgewaschen. Nach Trocknen im Vak. erhält man 90.1 g (75 %) O-Acetyl-L-homoserin Hydrochlorid.
Schmp.: 133 - 135 °C
¹H-NMR (DMSO): 2,01 (s, 3H), 2.16 (q, 2H), 3.94 (t, 1H), 4.15 (m, 2 H), 8.61 (s, 3H), 13.82 (s, 1H).

### N-Benzyloxycarbonyl-O-acetyl-L-homoserin (V)

### a) aus O-Acetyl-L-homoserin Hydrochlorid (VI)

19.8 g (0.10 mol) O-Acetyl-L-homoserin Hydrochlorid werden in 200 ml Wasser gelöst und bei 0°C mit 35.0 g (0.42 mol) Natriumhydrogencarbonat versetzt. Dann werden 19.2 g (0.11 mol) Benzylchlorformiat zugegeben und 3 h bei Raumtemperatur nachgerührt. Dann wird 2 mal mit je 100 ml Methylisobutylketon extrahiert, die wäßrige Phase mit konz. Salzsäure auf pH = 2 eingestellt und 2 mal mit je 150 ml Methylisobutylketon (MIBK) extrahiert. Die vereinigten organischen Phasen werden mit 50 ml gesättigter Natriumchloridlösung gewaschen und i. Vak. eingeengt. Man erhält 27.5 g (93 %) N-Benzyloxycarbonyl-O-acetyl-L-homoserin als farbloses Öl.
¹H-NMR (DMSO): 1.89 (m, 1H), 1.99 (s, 3H), 2.05 (m, 1H), 4.06 (m, 3H), 5.04 (s, 2H), 7.35 (m, 5 H), 7.63 (d, 1H), 12.70 (s, 1H).

### b) aus N-Benzyloxycarbonyl-L-homoserin (VIII)

1,27 g (5 mmol) N-Benzyloxycarbonyl-L-homoserin werden in 5 ml Essigsäure gelöst und mit 0,41 g (5 mmol) Natriumacetat versetzt. Dann gibt man 1.19 g (15 mmol) Acetylchlorid zu und rührt 1 h nach. Man entfernt die Essigsäure und das überschüssige Acetylchlorid i. Vak., nimmt den Rückstand in 30 ml Methylisobutylketon und 30 ml Wasser auf und stellt den pH-Wert mit 50%-iger Natronlauge auf 8. Die wäßrige Phase wird abgetrennt, mit Salzsäure auf pH = 2 gestellt und mit 30 ml Methylisobutylketon extrahiert. Nach Einengen der organischen Phase i. Vak erhält man 0.99 g (67 %) N-Benzyloxycarbonyl-O-acetyl-L-homoserin als farbloses Öl.

### O-Acetyl-L-homoserin-N-carboxyanhydrid (V)

In eine Suspension von 32.2 g (0.16 mol) O-Acetyl-L-homoserin Hydrochlorid in 180 ml Tetrahydrofuran werden 48.0 g (0.54 mol) Phosgen eingeleitet. Danach wird für 30 min zum Sieden erhitzt und das Lösungsmittel i. Vak. entfernt. Man erhält 29.2 g (98 %) O-Acetyl-L-homoserin-N-carboxyanhydrid als farbloses Öl.
¹H-NMR (DMSO): 1.97 (s, 3H), 2.07 (dd, 2H), 4.11 (t, 2 H), 4.54 (t, 1H), 9.12 (s, 1H).

### O-Acetyl-L-homoserin-p-cyanoanilid (IV)

Zu einer Suspension von 24.7 g (0.16 mol) Aminobenzonitril Hydrochlorid in 150 ml DMF werden 29.2 g (0.16 mol) O-Acetyl-L-homoserin-N-carboxyanhydrid gelöst in 100 ml DMF gegeben und 24 h bei Raumtemperatur gerührt. Nach Entfernen des Lösungsmittels i. Vak. erhält man ein Öl, das ohne weitere Reinigung weiter umgesetzt wird.
¹H-NMR (DMSO): 1.87 (s,3H), 2.22 (m,2H), 4.16 (m,2H), 4.23 (t,1H), 7.82 (d,2H), 7.92 (d,2H).

### N-Benzyloxycarbonyl-O-acetyl-L-homoserin-p-cyanoanilid (IV)

### a) aus N-Benzyloxycarbonyl-O-Acetyl-L-homoserin (V)

24.0 g (0.045 mol) N-Benzyloxycarbonyl-O-acetyl-L-homoserin werden in 75 ml Tetrahydrofuran gelöst , mit 6.2 ml (0.045 mol) Triethylamin versetzt und auf 0 °C abgekühlt. Dann gibt man 5.65 ml (0.045 mol) Pivaloylchlorid zu und läßt 15 min bei dieser Temperatur nachrühren. Nach Zugabe von 5.9 g (0.045 mol) p-Aminobenzonitril läßt man 24 h ohne Kühlung rühren. Nach Entfernen des Tetrahydrofurans i. Vak. nimmt man den Rückstand in 100 ml MIBK und 50 ml Wasser auf, stellt den pH-Wert mit konz. Salzsäure auf 2 und trennt die Phasen. Die organische Phase wird nochmals mit 50 ml Wasser gewaschen, mit weiteren 50 ml Wasser versetzt und mit 50%iger Natronlauge auf pH = 12 gestellt. Die organische Phase wird abgetrennt, mit 50 ml Wasser gewaschen und i. Vak. eingeengt. Der Rückstand wird mit 100 ml Methyl-tert.-butylether versetzt und der Niederschlag abgesaugt. Nach Trocknen i. Vak. erhält man 9.2 g (52 %) N-Benzyloxycarbonyl-O-acetyl-L-homoserin-p-cyanoanilid.
Schmp.: 76 - 78 °C
¹H-NMR (DMSO): 1.94 (s, 3 H), 1,96 (m, 1H), 2.06 (m, 1H), 4.09 (t, 2 H), 4.29 (m, 1H), 5.05 (s, 2H), 7.34 (m, 5H), 7,80 (m, 5H), 10.53 (s, 1H).

### b) aus O-Acetyl-L-homoserin-p-cyanoanilidhydrochlorid (IV)

17.5 g (80 mmol) O-Acetyl-L-homoserin-p-cyanoanilidhydrochlorid und 34 g (400 mmol) Natriumcarbonat in 150 ml Wasser werden bei 0 °C mit 18.1 g (104 mmol) Benzylchlorformiat versetzt und 14 h gerührt. Der ausgefallene Niederschlag wird abfiltriert, in 120 ml Methylisobutylketon und 50 ml Wasser gelöst und die Lösung mit konz. Salzsäure auf pH 2 gestellt. Die organische Phase wird abgetrennt, mit 30 ml Wasser gewaschen und i. Vak. eingeengt. Der Rückstand wird mit 200 ml Methyl-tert.-butylether verrührt und abgesaugt. Man erhält 16.9 g (53 %) N-Benzyloxycarbonyl-O-acetyl-L-homoserin-p-cyanoanilid.

### N-Benzyloxycarbonyl-L-homoserin-p-cyanoanilid (III)

14.0 g ( 0.035 mol) N-Benzyloxycarbonyl-O-acetyl-L-homoserin-p-cyanoanilid werden in 140 ml Methanol gelöst, mit 14 ml 25%-igem Ammoniak versetzt und 4 h auf 60 °C erhitzt. Man engt i. Vak. ein und versetzt den Rückstand mit 100 ml Methyl-tert.-butylether. Der Niederschlag wird abgesaugt und i. Vak. getrocknet. Man erhält 8.7 g N-Benzyloxycarbonyl-L-homoserin-p-cyanoanilid.
Schmp.: 125 - 128°C
¹H-NMR (DMSO): 1.78 (m, 1H), 1.86 (m, 1H), 4.49 (dt, 2H), 4.27 (m, 1H), 4.61 (t, 1H), 5.03 (s, 2H), 7.37 (m, 5H), 7.61 (d, 1H), 7.79 (dd, 4H), 10.45 (s, 1H).

### 3-Benzyloxycarbonyl-amino-1-(4-cyanophenyl)-2-oxopyrrolidin (I)

### a) mit Mesylchlorid / Triethylamin

Zu 63.6 g (0,18 mol) N-Benzyloxycarbonyl-L-homoserin-p-cyanoanilid in 600 ml Tetrahydrofuran werden bei -10 °C 31,0 g (0,27 mol) Mesylchlorid zugegeben und während 15 min 37,4 ml (0.27 mol) Triethylamin so zugetropft, daß die Temperatur unter -5 °C bleibt. Man läßt 1 Stunde bei -10 °C nachrühren, gibt dann 54 g 50%-ige Natronlauge zu und läßt 12 Stunden bei Raumtemperatur rühren. Dann gibt man 1 l Wasser zu und stellt den pH-Wert mit konz. Salzsäure auf pH = 8. Das nach Abziehen des Tetrahydrofurans i. Vak. ausgefallene Produkt wird abgesaugt. Zur weiteren Reinigung wird das Rohprodukt mit 1 l MIBK verrührt. Nach Trocknen erhält man 38.6 g (64 % d. Th.) 3-Benzyloxycarbonyl-amino-1-(4-cyanophenyl)-2-oxo-pyrrolidin.
Schmp: 192 - 196 °C
1H-NMR (DMSO): 2.02 (m, 1H), 2.41 (m, 1H), 3.78 (m, 1H), 3.84 (m, 1H), 4.47 (m, 1H), 5.07 (s, 2H), 7.33, 7.38 (m, 5H), 7.77 (d, 1H), 7.88 (dd, 4H).

### b) mit Tosylchlorid / Pyridin

Zu 1.0 g (2.8 mmol) N-Benzyloxycarbonyl-L-homoserin-p-cyanoanilid in 10 ml Pyridin werden 0.59 g (3.1 mmol) Tosylchlorid gegeben und 1 h bei Raumtemperatur gerührt. Das Pyridin wird i. Vak. abgezogen, der Rückstand in 20 ml Tetrahydrofuran gelöst und mit 1 g 50 %-iger NaOH versetzt. Man läßt über Nacht rühren, gibt dann 20 ml Wasser zu und zieht das Tetrahydrofuran i. Vak. ab. Der Niederschlag wird abgesaugt und mit MIBK gewaschen. Man erhält 0.44 g (47 %) 3-Benzyloxycarbonyl-amino-1-(4-cyanophenyl)-2-oxopyrrolidin.

### Herstellung des 3-Amino-1-(4-cyanophenyl)-2-oxopyrrolidinhydrochlorid (I)

50 g (0.15 mol) 3-Benzyloxycarbonyl-amino-1-(4-cyanophenyl)-2-oxo-pyrrolidin werden in 600 ml Methanol suspendiert und mit 14.1 ml 36%-iger Salzsäure versetzt. Nach Zugabe von 2.5 g 5 % Palladium auf A-Kohle wird bei 45 °C Wasserstoff durchgeleitet. Nach 40 min wird abgekühlt, der Katalysator abfiltriert und das Filtrat zur Trockene eingeengt. Nach Umkristallisation aus Methanol/Isopropanol erhält man 28.5 g (80 %) 3-Amino-1-(4-cyanophenyl)-2-oxo-pyrrolidin Hydrochlorid.
Schmp: 256 - 258 °C (Zers.)
1H-NMR (DMSO): 2.20 (m, 1H), 2.55 (m, 1H), 3.87 (dt, 1H), 3.97 (t, 1H); 4.27 (dd, 1H), 7.91 (s, 4H), 8.89 (s, 3H).

### Herstellung von 3-Benzyloxycarbonyl-amino-1-(4-cyanophenyl)-2-oxo-pyrrolidin (I) aus O-Acetyl-L-homoserinhydrochlorid (VI) ohne Isolierung der Zwischenstufen

Zu einer Lösung von 1600 g (19.04 mol) Natriumhydrogencarbonat in 9.3 l Wasser werden 1883 g (9.52 mol) O-Acetyl-L-homoserin Hydrochlorid gegeben und auf 5 °C abgekühlt. Dann werden 1823 g (10.47 mol) Benzylchloroformiat zugegeben und der pH-Wert durch Zugabe von 20%-iger Natriumcarbonatlösung auf 7.5 gehalten. Es wird 2 h bei 15°C nachgerührt und dann mit 20 l Methylisobutylketon extrahiert. Die wäßrige Phase wird mit konz. Salzsäure auf pH = 2.0 gestellt und zweimal mit insgesamt 25 l Methylisobutylketon extrahiert. Die vereinigten organischen Phasen werden mit 3.5 l Wasser gewaschen und i. Vak. eingeengt. Der Rückstand wird in 19,5 l Methylisobutylketon aufgenommen, mit 856 g (8.47 mol) Triethylamin versetzt und auf 0 °C abgekühlt. Dann werden 1042 g (8.47 mol) Pivaloylchlorid zugegeben und 20 min nachgerührt. Nach Zugabe von 1000 g (8.47 mol) p-Aminobenzonitril wird 36 h bei Raumtemperatur gerührt. Dann werden 10 l Wasser zugegeben und mit konz. Salzsäure auf pH = 2.0 gestellt. Die organische Phase wird abgetrennt, mit 10 l Wasser versetzt und mit 50%-iger Natronlauge auf pH = 11 gestellt. Nach Waschen mit 3 l Wasser wird die organische Phase i. Vak. bis auf ca. 3.5 kg eingeengt. Der Rückstand wird in 25 l Methanol gelöst und mit 7.5 l 25%-igen Ammoniak versetzt. Danach wird 6 h auf 60 °C erwärmt und anschließend i. Vak. eingeengt. Der Rückstand wird durch Abdestillieren von dreimal je 7.5 l Methylisobutylketon entwässert, in 25 l Tetrahydrofuran aufgenommen, auf -5°C abgekühlt und mit 1522 g (13.28 mol) Mesylchlorid versetzt. Dann läßt man während 45 min 1341 g (13.28 mol) Triethylamin zulaufen, rührt 1 h nach, gibt dann 2300 g 50%-ige Natronlauge zu und rührt 1 h nach. Zum Reaktionsgemisch werden dann 3 l Wasser gegeben und der pH-Wert mit konz. Salzsäure auf 7 gestellt. Der nach Entfernen des Tetrahydrofurans i. Vak. erhaltene Feststoff wird mit 14 l Wasser verrührt, abgesaugt und mit Wasser gewaschen. Das Rohprodukt wird mit 19 l Methylisobutylketon bei 50 °C verrührt und abgesaugt. Nach Trocknen erhält man 1401 g (44 %) 3-Benzyloxycarbonyl-amino-1-(4-cyanophenyl)-2-oxo-pyrrolidin.

### N-Boc-O-acetyl-L-homoserin (V)

Zu einer Suspension von 9.9 g (50 mmol) O-Acetyl-L-homoserin Hydrochlorid und 21.8 g (100 mmol) Boc-anhydrid in 100 ml Tetrahydrofuran werden innerhalb von 1 h 10.1 g (100 mmol) Triethylamin zugetropft und 12 h bei Raumtemperatur gerührt. Nach zugaben von 100 ml Wasser wird das Tetrahydrofuran i. Vak. abdestilliert, mit konz. Salzsäure der pH-Wert auf 2 gestellt und 2 mal mit je 100 ml Methylisobutylketon extrahiert. Nach Einengen der Lösung i. Vak. erhält man 12.1 g (92 %) N-Boc-O-acetyl-L-homoserin als farbloses Öl.
1H-NMR (DMSO): 1.38 (s, 9H), 1.84 (m, 1H), 1.99 (m, 1H), 1.99 (s, 3H), 4.00 (m, 3H), 7.11 (d, 1H).

### N-Trifluoracetyl-O-acetyl-L-homoserin (V)

Zu einer Suspension von 9.9 g (50 mmol) O-Acetyl-L-homoserin Hydrochlorid und 7,8 g (55 mmol) Trifluoracetylessigsäureethylester in 200 ml Ethanol werden 7,6 g (55 mol) Kaliumcarbonat gegeben und 14 h Gerührt. Danach werden 10.8 g (110 mmol) 37%-ige Salzsäure und 200 ml Wasser zugegeben. Das nach Abdestillieren des Ethanols ausgefallene Produkt wird abgesaugt, mit Wasser gewaschen und getrocknet. Man erhält 5,8 g (45 %) N-Trifluoracetyl-O-acetyl-L-homoserin.
Schmp.: 134 - 136 °C
1H-NMR (DMSO): 1.99 (s, 3H), 2.04 (m, 1H), 2.19 (m, 1H), 4.06 (t, 2H), 4.37 (m, 1H), 9.73 (d, 1H), 13.08 (s, 1H).

## Patentansprüche

1. Verfahren zur Herstellung von γ-Lactamen der allgemeinen Formel I und deren Salze 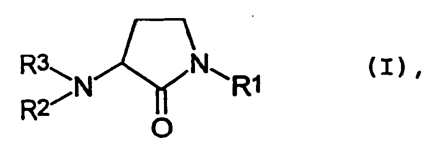 worin
R¹ bedeuten kann H, (C₁-C₈)-Alkyl, (C₂-C₈)-Alkenyl, (C₂-C₈)-Alkinyl, (C₂-C₈) -Alkoxyalkyl, (C₁-C₈)-Acyl,
welche gegebenenfalls linear oder verzweigt vorliegen sowie einfach oder mehrfach mit Halogenen, N-, O-, P-, S-atomhaltigen Resten substituiert sein können, (C₃-C₇)-Cycloalkyl, welche gesättigt oder ungesättigt sowie einfach oder mehrfach mit linear oder verzweigten (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein und/oder im Ring Heteroatome wie N, O, P, S enthalten können,
Aryl, wie Phenyl oder Naphthyl, Aralkyl, wie Benzyl oder Phenethyl,
Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die eben genannten Cyclen gegebenenfalls einfach oder mehrfach mit linear oder verzweigten (C₁-C₈)-Alkyl, (C₂-C₈) -Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein können,
N-verknüpfter Aminosäure- oder Peptidrest,
R² bedeuten kann H, (C₁-C₈)-Alkyl, (C₂-C₈)-Alkenyl, (C₂-C₈)-Alkinyl, (C₂-C₈)-Alkoxyalkyl, welche gegebenenfalls linear oder verzweigt vorliegen sowie einfach oder mehrfach mit Halogenen, N-, O-, P-, S-atomhaltigen Resten substituiert sein können, (C₃-C₇)-Cycloalkyl, welche gesättigt oder ungesättigt sowie einfach oder mehrfach mit linear oder verzweigten (C₁-C₈)-Alkyl, (C₂-C₈)-Alkenyl, (C₂-C₈)-Alkinyl, (C₁-C₈)-Acyl, (C₁-C₈)-Alkoxy, (C₂-C₈)-Alkoxyalkyl, mit Halogenen, N-, O-, P-, S-atomhaltigen Resten substituiert sein und/oder im Ring Heteroatome wie N, O, P, S enthalten können,
Aryl, wie Phenyl oder Naphthyl, Aralkyl, wie Benzyl oder Phenethyl,
Heteroaryl, wie Furyl, Pyrrolyl, Pyridyl, Heteroaralkyl, wie Furfuryl, Pyrrolylmethyl, Pyridylmethyl, Furylethyl, Pyrrolylethyl, Pyridylethyl, wobei die eben genannten Cyclen gegebenenfalls einfach oder mehrfach mit linear oder verzweigten (C₁-C₈)-Alkyl, (C₂-C₈)-Alkenyl, (C₂-C₈)-Alkinyl, (C₁-C₉)-Acyl, (C₁-C₈)-Alkoxy, (C₂-C₈)-Alkoxyalkyl, mit Halogenen, N-, O-, P-, S-atomhaltigen Resten substituiert sein können,
R³ bedeuten kann H, ClCO, (C₁-C₈) -Acyl, welcher gegebenenfalls linear oder verzweigt vorliegen kann, ein C-verknüpfter Aminosäure- oder ein Peptidrest oder eine gängige Peptidschutzgruppe wie z. B. Formyl, Carbamoyl, Benzyloxycarbonyl, tert.-Butyloxycarbonyl, Allyloxycarbonyl, Trifluoracetyl,
**dadurch gekennzeichnet,**
**daß** man Derivate der allgemeinen Formel II 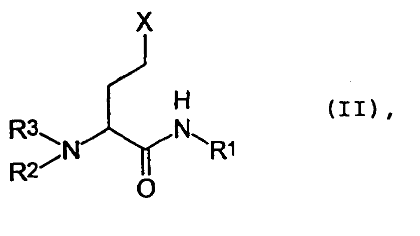 in der R¹, R², R³ die oben angegebene Bedeutung besitzen und X einen Sulfonsäureester darstellt, zu Verbindungen der allgemeinen Formel I cyclisiert.

2. Verfahren nach Anspruch 1,
**dadurch gekennzeichnet,**
**daß** man unter basischen Bedingungen cyclisiert.

3. Verfahren nach Anspruch 2,
**dadurch gekennzeichnet,**
**daß** man Natriumhydroxidlösung als Base einsetzt.

4. Verfahren nach einem oder mehreren der Ansprüche 1-3,
**dadurch gekennzeichnet,**
**daß** X ein Sulfonsäureester ist.

5. Verfahren nach Anspruch 4,
**dadurch gekennzeichnet,**
**daß** X die OSO₃Me-Gruppe ist.

6. Verfahren nach einem oder mehreren der Ansprüche 1-5,
**dadurch gekennzeichnet,**
**daß** man als Verbindung II eine Verbindung einsetzt, bei der R¹ p-Cyanophenyl oder p-Carbamoylphenyl, R² H und R³ Benzyloxycarbonyl ist.

7. Verfahren nach einem oder mehreren der Ansprüche 1-6,
**dadurch gekennzeichnet,**
**daß** man die Cyclisierung bei Temperaturen von -20 bis 100 °C betreibt.

8. Verfahren nach einem oder mehreren der Ansprüche 1-7,
**dadurch gekennzeichnet,**
**daß** man die Verbindung der allgemeinen Formel II ohne vorherige Isolierung zur Verbindung der allgemeinen Formel I cyclisiert.

9. Verfahren nach einem oder mehreren der Ansprüch 1-8,
**dadurch gekennzeichnet,**
**daß** man Verbindungen der allgemeinen Formel II aus Derivaten der allgemeinen Formel III 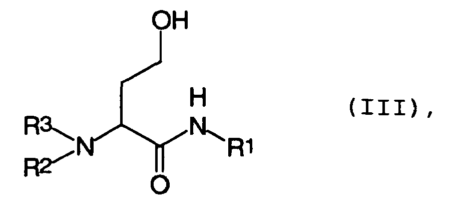 worin R¹, R², R³ die oben angegebene Bedeutung besitzen, durch Sulfonsäureesterbildung herstellt.

10. Verfahren nach Anspruch 9,
**dadurch gekennzeichnet,**
**daß** man die Verbindung der allgemeinen Formel III nicht zwischenisoliert.

11. Verfahren nach einem der Ansprüche 9 oder 10,
**dadurch gekennzeichnet,**
**daß** man die Verbindung der allgemeinen Formel III aus Derivaten der allgemeinen Formel IV 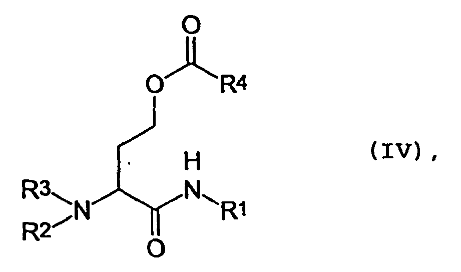 worin R¹, R², R³ die oben angegebene Bedeutung besitzen und R⁴ steht für (C₁-C₈)-Alkyl, (C₂-C₈)-Alkenyl, (C₁-C₈)-Alkoxy, (C₂-C₈)-Alkenyloxy, welche ggf. linear oder verzweigt vorliegen und ggf. mit einem oder mehreren Halogenatomen substituiert sind, Aryl, wie Phenyl oder Naphthyl, Aralkyl, wie Benzyl oder Phenethyl, Arylalkyloxy, wie Benzyloxy, herstellt.

12. Verfahren nach Anspruch 11,
**dadurch gekennzeichnet,**
**daß** man die Verbindung der Formel IV durch Aminolyse in Verbindungen der allgemeinen Formel III überführt.

13. Verfahren nach Anspruch 11 und/oder 12,
**dadurch gekennzeichnet,**
**daß** man die Verbindungen der allgemeinen Formel IV aus Derivaten der allgemeinen Formel V 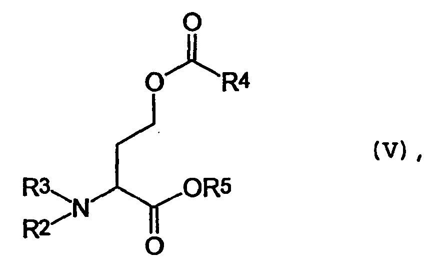 worin R² , R³, R⁴ die oben angegebene Bedeutung besitzen und R⁵ für H steht oder worin R³ und R⁵ über eine C=O-Gruppe zu einem Ring verbunden sind, herstellt.

14. Verfahren nach Anspruch 13,
**dadurch gekennzeichnet,**
**daß** man Verbindungen der allgemeinen Formel V, in der R⁵ für H steht, vor der Umsetzung zu Verbindungen der allgemeinen Formel IV durch Säurechloride aktiviert.

15. Verfahren nach Anspruch 13 und/oder 14,
**dadurch gekennzeichnet,**
**daß** man ein Säureadditionssalz der Verbindung der allgemeinen Formel VI 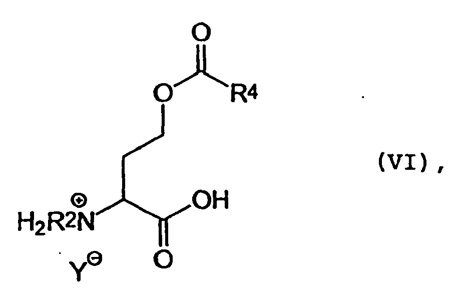 in der R², R⁴ die oben angegebene Bedeutung hat und Y ^{⊖} die korrespondierende Base einer anorganischen Säure ist, mit einem sich von R³ ableitenden Acylierungsreagenz zur Verbindung der allgemeinen Formel V umsetzt.

16. Verfahren nach Anspruch 15,
**dadurch gekennzeichnet,**
**daß** man als Acylierungsreagenz Phosgen einsetzt.

17. Verfahren nach Anspruch 15,
**dadurch gekennzeichnet,**
**daß** man ein Acylierungsreagenz der Gruppe der organischen Anhydride, aktivierten Ester, Halokohlensäureester einsetzt.

18. Verfahren nach Anspruch 17,
**dadurch gekennzeichnet,**
**daß** man die Acylierung in wäßriger Lösung bei einem pH-Wert von 4-9 durchführt.

19. Verfahren nach Anspruch 17,
**dadurch gekennzeichnet,**
**daß** man die Acylierung in einem organischen Lösungsmittel in Gegenwart einer tertiären Base durchführt.

20. Verfahren nach einem oder mehreren der Ansprüche 15-19,
**dadurch gekennzeichnet,**
**daß** man das Säureadditionssalz der allgemeinen Formel VI durch Umsetzung von Verbindungen der allgemeinen Formel VII 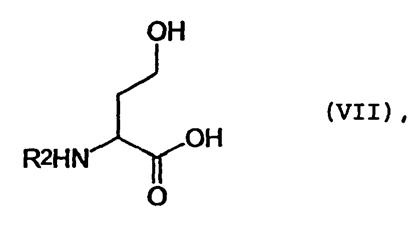 in der R² die oben angegebene Bedeutung hat,
in einem Gemisch aus R⁴COOH und R⁴COCl oder R⁴COBr, wobei R⁴ die oben angegebene Bedeutung hat, herstellt.

21. Verfahren nach Anspruch 20,
**dadurch gekennzeichnet,**
**daß** man zuerst die Carbonsäure und das Carbonsäurehalogenid mischt und anschließend die Verbindung der allgemeinen Formel VII zu dieser Mischung hinzufügt.

22. Verfahren nach einem oder mehreren der Ansprüche 20 oder 21,
**dadurch gekennzeichnet,**
**daß** man bei einer Temperatur von -20 °C bis 50 °C arbeitet.

23. Verfahren nach einem oder mehreren der Ansprüche 13 oder 14,
**dadurch gekennzeichnet,**
**daß** man eine Verbindung der allgemeinen Formel VIII 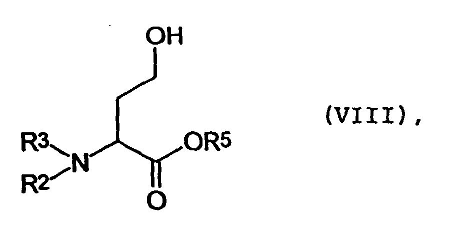 in der die Reste R², R³ die oben angegebene Bedeutung innehaben und R⁵ für H steht, mit dem Reagenz R⁴COZ, worin R⁴ die oben angegebene Bedeutung besitzt und Z einen aktivierenden Rest darstellt, zur Verbindung der allgemeinen Formel V umsetzt.

24. Verfahren nach Anspruch 23,
**dadurch gekennzeichnet,**
**daß** im Falle von Z = Cl oder Br die korrespondierende Carbonsäure als Lösungsmittel verwendet wird.

25. Verfahren nach Anspruch 24,
**dadurch gekennzeichnet,**
**daß** man dem Reaktionsgemisch als Base das Natriumsalz der korrespondierenden als Lösungsmittel eingesetzten Carbonsäure zusetzt.

26. Verbindungen der allgemeinen Formel V und deren Salze 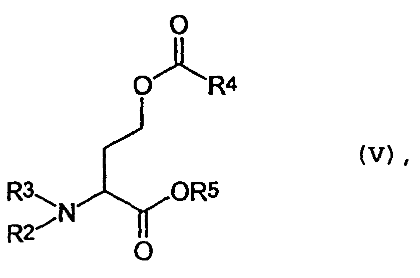 worin R² als H, R³ Benzyloxycarbonyl oder tert.-Butyloxycarbonyl oder Trifluoracetyl , R⁴ als Methyl und R⁵ als H steht.

27. Verbindungen der allgemeinen Formel V mit R² als H, R⁴ als Methyl und worin R³ und R⁵ über eine C=O-Gruppe zu einem Ring verbunden sind.

28. Verbindungen der allgemeinen Formel IV und deren Salze 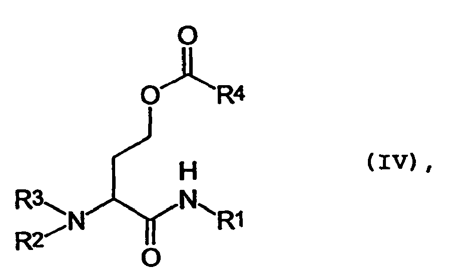 worin R¹, die Bedeutung gemäß Anspruch 1 und
R² als H,
R³ als Benzyloxycarbonyl oder tert.-Butyloxycarbonyl oder Trifluoracetyl und R⁴ als Methyl steht.

29. Verbindungen der allgemeinen Formel IV mit R² als H,
R³ Benzyloxycarbonyl, R⁴ als Methyl und R¹ als
p-Cyanophenyl oder p-Carbamoylphenyl

30. Verbindungen der allgemeinen Formel II und deren Salze 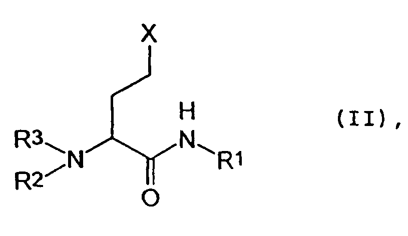 in der R¹, R², R³ die Bedeutung gemäß Anspruch 1 besitzen und X einen Sulfonsäureester darstellt.

31. Verbindungen der allgemeinen Formel II mit R² als H, R³ Benzyloxycarbonyl oder tert.-Butyloxycarbonyl oder Trifluoracetyl und X als Sulfonsäureester.

32. Verbindungen der allgemeinen Formel II mit R² als H, R³ Benzyloxycarbonyl, X als OSO₃Me-Gruppe und R¹ als p-Cyanophenyl oder p-Carbamoylphenyl

33. Verwendung der Verbindungen gemäß Anspruch 26, 29 und 32 zur Herstellung von Intermediaten für bioaktive Wirkstoffe.

## Claims

1. Process for the preparation of γ-lactams of the general formula I and their salts 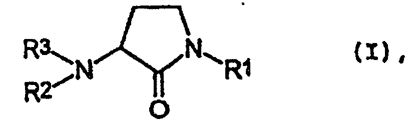 wherein
R¹ may represent H, (C₁-C₈) -alkyl, (C₂-C₈) -alkenyl, (C₂-C₈) -alkynyl, (C₂-C₈) -alkoxyalkyl, (C₁-C₈) -acyl, which are each optionally linear or branched and may be mono- or poly-substituted by halogens, by radicals having N, O, P, S atoms,
(C₃-C₇) -cycloalkyl, which may be saturated or unsaturated and mono- or poly-substituted by linear or branched (C₁-C₈)-alkyl, (C₂-C₈)-alkenyl, (C₂-C₈) -alkynyl, (C₁-C₈) -acyl, (C₁ -C₈)-alkoxy, (C₂-C₈) -alkoxyalkyl, by halogens, by radicals having N, O, P, S atoms, and/or may have heteroatoms such as N, O, P, S in the ring,
aryl, such as phenyl or naphthyl, aralkyl, such as benzyl or phenethyl,
heteroaryl, such as furyl, pyrrolyl, pyridyl, heteroaralkyl, such as furfuryl, pyrrolylmethyl, pyridylmethyl, furylethyl, pyrrolylethyl, pyridylethyl, wherein the cycles just mentioned may optionally be mono- or poly-substituted by linear or branched (C₁-C₈)-alkyl, (C₂-C₈)-alkenyl, (C₂-C₈)-alkynyl, (C₁-C₈)-acyl, (C₁-C₈) -alkoxy, (C₂-C₈)-alkoxyalkyl, by halogens, by radicals having N, O, P, S atoms,
N-bonded amino acid or peptide residue,
R²may represent H, (C₁-C₈) -alkyl, (C₂-C₈) -alkenyl, (C₂-C₈) -alkynyl, (C₂-C₈)-alkoxyalkyl, which are each optionally linear or branched and may be mono- or poly-substituted by halogens, by radicals having N, O, P, S atoms,
(C₃-C₇)-cycloalkyl, which may be saturated or unsaturated and mono- or poly-substituted by linear or branched (C₁-C₈) -alkyl, (C₂-C₈) -alkenyl, (C₂-C₈) -alkynyl, (C₁-C₈) -acyl, (C₁-C₈) -alkoxy, (C₂-C₈) -alkoxyalkyl, by halogens, by radicals having N, O, P, S atoms, and/or may have heteroatoms such as N, O, P, S in the ring,
aryl, such as phenyl or naphthyl, aralkyl, such as benzyl or phenethyl,
heteroaryl, such as furyl, pyrrolyl, pyridyl,
heteroaralkyl, such as furfuryl, pyrrolylmethyl, pyridylmethyl, furylethyl, pyrrolylethyl, pyridylethyl, wherein the cycles just mentioned may optionally be mono- or poly-substituted by linear or branched (C₁-C₈)-alkyl, (C₂-C₈)-alkenyl, (C₂-C₈)-alkynyl, (C₁-C₈)-acyl, (C₁-C₈)-alkoxy, (C₂-C₈)-alkoxyalkyl, by halogens, by radicals having N, O, P, S atoms,
R³ may represent H, ClCO, (C₁-C₈)-acyl, which may optionally be linear or branched, a C-bonded amino acid residue or a peptide residue or a conventional peptide-protecting group such as for example formyl, carbamoyl, benzyloxycarbonyl, tert-butyloxycarbonyl, allyloxycarbonyl, trifluoroacetyl,
**characterized in that**
derivatives of the general formula II 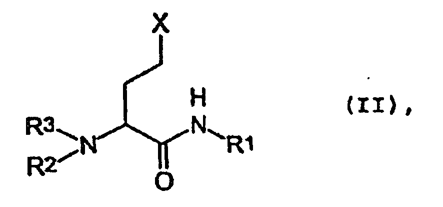 wherein R¹, R², R³ are as defined above and X represents a sulfonic acid ester, are cyclised to form compounds of the general formula I.

2. Process according to Claim 1,
**characterized in that**
the cyclisation is carried out under basic conditions.

3. Process according to Claim 2, **characterized in that** sodium hydroxide solution is used as a base.

4. Process according to one or more of Claims 1-3,
**characterized in that**
X is a sulfonic acid ester.

5. Process according to Claim 4,
**characterized in that**
X is an OSO₃Me group.

6. Process according to one or more of Claims 1-5,
**characterized in that**
compound II is a compound wherein R¹ is p-cyanophenyl or p-carbamoylphenyl, R² is H and R³ is benzyloxycarbonyl.

7. Process according to one or more of Claims 1-6,
**characterized in that**
the cyclisation is carried out at temperatures of from -20 to 100°C.

8. Process according to one or more of Claims 1-7,
**characterized in that**
the compound of the general formula II is cyclised to form the compound of the general formula I without being isolated beforehand.

9. Process according to one or more of Claims 1-8,
**characterized in that**
compounds of the general formula II are prepared from derivatives of the general formula III 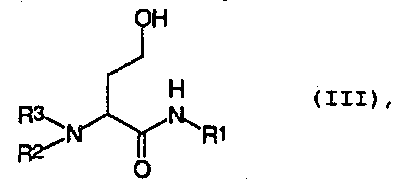 wherein R¹, R², R³ are as defined above, by sulfonic ester formation.

10. Process according to Claim 9,
**characterized in that**
the compound of the general formula III is not isolated as an intermediate.

11. Process according to Claim 9 or Claim 10,
**characterized in that**
the compound of the general formula III is prepared from derivatives of the general formula IV 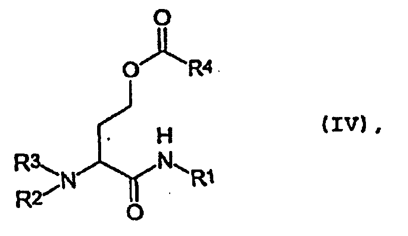 wherein R¹, R² , R³ are as defined above and R⁴ represents (C₁-C₈) -alkyl, (C₂-C₈)-alkenyl, (C₁-C₈) - alkoxy, (C₂-C₈)-alkenyloxy, which are optionally linear or branched and are optionally substituted by one or more halogen atoms, aryl, such as phenyl or naphthyl, aralkyl, such as benzyl or phenethyl, arylalkyloxy, such as benzyloxy.

12. Process according to Claim 11,
**characterized in that**
the compound of the formula IV is converted into compounds of the general formula III by aminolysis.

13. Process according to Claim 11 and/or 12,
**characterized in that**
the compounds of the general formula IV are prepared from derivatives of the general formula V 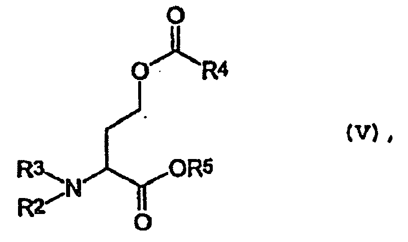 wherein R², R³, R⁴ are as defined above and R⁵ represents H or wherein R³ and R⁵ are bonded together by a C=O group to form a ring.

14. Process according to Claim 13,
**characterized in that**
compounds of the general formula V wherein R⁵ represents H are activated by means of acid chlorides before being reacted to form compounds of the general formula IV.

15. Process according to Claim 13 and/or 14,
**characterized in that**
an acid addition salt of the compound of the general formula VI 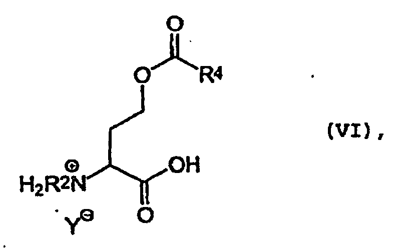 wherein R², R⁴ are as defined above and Y^{⊖} is the corresponding base of an inorganic acid, is reacted with an acylating reagent derived from R³ to form the compound of the general formula V.

16. Process according to Claim 15,
**characterized in that**
phosgene is used as the acylating reagent.

17. Process according to Claim 15,
**characterized in that**
an acylating reagent from the group of the organic anhydrides, activated esters and halocarbonic esters is used.

18. Process according to Claim 17,
**characterized in that**
the acylation is carried out in aqueous solution at a pH of 4-9.

19. Process according to Claim 17,
**characterized in that**
the acylation is carried out in an organic solvent in the presence of a tertiary base.

20. Process according to one or more of Claims 15 to 19,
**characterized in that**
the acid addition salt of the general formula VI is prepared by reaction of the compounds of the general formula VII 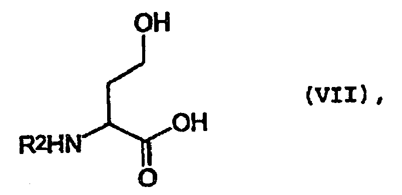 wherein R² is as defined above, in a mixture of R⁴COOH and R⁴COCl or R⁴COBr, wherein R⁴ is as defined above.

21. Process according to Claim 20,
**characterized in that**
first the carboxylic acid and the carboxylic acid halide are mixed and then the compound of the general formula VII is added to that mixture.

22. Process according to one or more of Claims 20 or 21,
**characterized in that**
a temperature of from -20°C to 50°C is employed.

23. Process according to one or more of Claims 13 or 14,
**characterized in that**
a compound of the general formula VIII 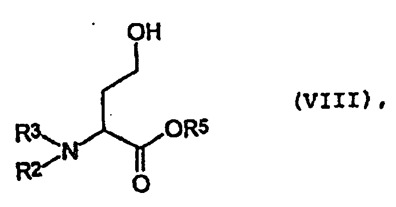 wherein the radicals R², R³ are as defined above and R⁵ represents H, is reacted with the reagent R⁴COZ, wherein R⁴ is as defined above and Z represents an activating radical, to form the compound of the general formula V.

24. Process according to Claim 23,
**characterized in that**
when Z = Cl or Br, the corresponding carboxylic acid is used as solvent.

25. Process according to Claim 24,
**characterized in that**
the sodium salt of the corresponding carboxylic acid used as solvent is added to the reaction mixture as base.

26. Compounds of the general formula V and their salts 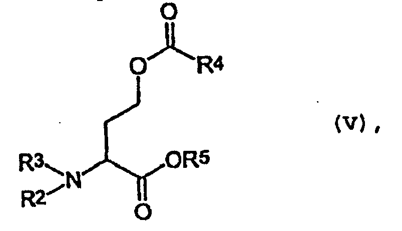 wherein R² is H, R³ is benzyloxycarbonyl or tert-butyloxycarbonyl or trifluoroacetyl, R⁴ is methyl and R⁵ is H.

27. Compounds of the general formula V with R² as H, R⁴ as methyl and wherein R³ and R⁵ are bonded via a C=O group to form a ring.

28. Compounds of the general formula IV and their salts 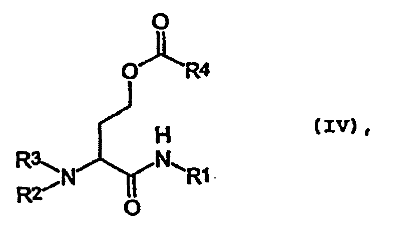 wherein R¹ is as defined in claim 1 and R² is H, R³ is benzyloxycarbonyl or tert-butyloxycarbonyl or trifluoroacetyl and R⁴ is methyl.

29. Compounds of the general formula IV with R² as H, R³ benzyloxycarbonyl, R⁴ as methyl and R¹ as p-cyanophenyl or p-carbamoylphenyl.

30. Compounds of the general formula II and their salts 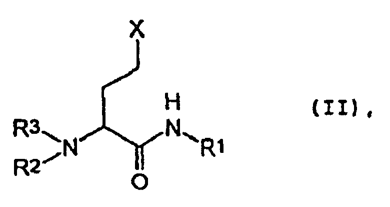 wherein R¹, R², R³ are as defined in Claim 1 and X is a sulfonic acid ester.

31. Compounds of the general formula II with R² as H, R³ benzyloxycarbonyl or tert-butyloxycarbonyl or trifluoroacetyl and X as sulfonic acid ester.

32. Compounds of the general formula II with R² as H, R³ benzyloxycarbonyl, X as OSO₃Me group and R¹ as p-cyanophenyl or p-carbamoylphenyl.

33. Use of compounds according to Claim 26, 29 and 32 for preparing intermediates for bioactive compounds.

## Revendications

1. Procédé de préparation de γ-lactames de formule générale I et de leurs sels 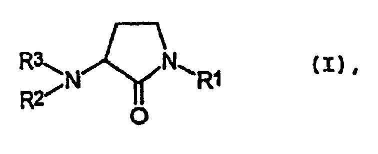 dans laquelle :
R¹ peut représenter :
un atome d'hydrogène, un groupe alkyle en (C₁-C₈), un groupe alcényle en (C₂-C₈), un groupe alcynyle en (C₂-C₈), un groupe alcoxyalkyle en (C₂-C₈), un groupe acyle en (C₁-C₈), qui peuvent être éventuellement linéaires ou ramifiés et être monosubstitués ou polysubstitués par des atomes d'halogène, des radicaux renfermant un atome d'azote, un atome d'oxygène, un atome de phosphore ou un atome de soufre,
un groupe cycloalkyle en (C₃-C₇), qui peut être saturé ou insaturé et mono substitué ou polysubstitué par un groupe alkyle en (C₁-C₈), un groupe alcényle en (C₂-C₈), un groupe alcynyle en (C₂-C₈), un groupe acyle en (C₁-C₈), un groupe alcoxy en (C₁-C₈), un groupe alcoxyalkyle en (C₂-C₈), linéaires ou ramifiés, par des atomes d'halogène, des radicaux renfermant un atome d'azote, un atome d'oxygène, un atome de phosphore ou un atome de soufre, et/ou peuvent renfermer dans le noyau des hétéroatomes tels qu'un atome d'azote, un atome d'oxygène, un atome de phosphore, un atome de soufre,
un groupe aryle, tel qu'un groupe phényle ou un groupe naphtyle, un groupe aralkyle, tel qu'un groupe benzyle ou un groupe phénéthyle,
un groupe hétéroaryle, tel qu'un groupe furyle, un groupe pyrrolyle, un groupe pyridyle, un groupe hétéroaralkyle, tel qu'un groupe furfuryle, un groupe pyrrolylméthyle, un groupe pyridylméthyle, un groupe furyléthyle, un groupe pyrrolyléthyle, un groupe pyridyléthyle, où les cycles mentionnés ci-dessus peuvent être éventuellement monosubstitués ou polysubstitués par un groupe alkyle en (C₁-C₈), un groupe alcényle en (C₂-C₈), un groupe alcynyle en (C₂-C₈), un groupe acyle en (C₁-C₈), un groupe alcoxy en (C₁-C₈), un groupe alcoxyalkyle en (C₂-C₈), linéaires ou ramifiés, par des atomes d'halogène, des radicaux renfermant un atome d'azote, un atome d'oxygène, un atome de phosphore ou un atome de soufre,
un radical acide aminé ou peptidique lié à un atome d'azote,
R² peut représenter :
un atome d'hydrogène, un groupe alkyle en (C₁-C₈), un groupe alcényle en (C₂-C₈), un groupe alcynyle en (C₂-C₈), un groupe alcoxyalkyle en (C₂-C₈), qui peuvent être éventuellement linéaires ou ramifiés et être monosubstitués ou polysubstitués par des atomes d'halogène, des radicaux renfermant un atome d'azote, un atome d'oxygène, un atome de phosphore ou un atome de soufre,
un groupe cycloalkyle en (C₃-C₇), qui peut être saturé ou insaturé et monosubstitué ou polysubstitué par un groupe alkyle en (C₁-C₈), un groupe alcényle en (C₂-C₈), un groupe alcynyle en (C₂-C₈), un groupe acyle en (C₁-C₈), un groupe alcoxy en (C₁-C₈), un groupe alcoxyalkyle en (C₂-C₈), linéaires ou ramifiés, par des atomes d'halogène, des radicaux renfermant un atome d'azote, un atome d'oxygène, un atome de phosphore ou un atome de soufre, et/ou peuvent renfermer dans le noyau des hétéroatomes tels qu'un atome d'azote, un atome d'oxygène, un atome de phosphore, un atome de soufre,
un groupe aryle, tel qu'un groupe phényle ou un groupe naphtyle, un groupe aralkyle, tel qu'un groupe benzyle ou un groupe phénéthyle,
un groupe hétéroaryle, tel qu'un groupe furyle, un groupe pyrrolyle, un groupe pyridyle, un groupe hétéroaralkyle, tel qu'un groupe furfuryle, un groupe pyrrolylméthyle, un groupe pyridylméthyle, un groupe furyléthyle, un groupe pyrrolyléthyle, un groupe pyridyléthyle, où les cycles mentionnés ci-dessus peuvent être éventuellement monosubstitués ou polysubstitués par un groupe alkyle en (C₁-C₈), un groupe alcényle en (C₂-C₈), un groupe alcynyle en (C₂-C₈), un groupe acyle en (C₁-C₈), un groupe alcoxy en (C₁-C₈), un groupe alcoxyalkyle en (C₂-C₈), linéaires ou ramifiés, par des atomes d'halogène, des radicaux renfermant un atome d'azote, un atome d'oxygène, un atome de phosphore ou un atome de soufre,
R³ peut représenter un atome d'hydrogène, un groupe CICO, un groupe acyle en (C₁-C₈), qui peut être éventuellement linéaire ou ramifié, un radical acide aminé ou peptidique lié à un atome de carbone ou un groupe protecteur de peptide habituel, tel que, par exemple, un groupe formyle, un groupe carbamoyle, un groupe benzyloxycarbonyle, un groupe tert-butyloxycarbonyle, un groupe allyloxycarbonyle, un groupe trifluoroacétyle,
**caractérisé en ce que**
des dérivés de formule générale II 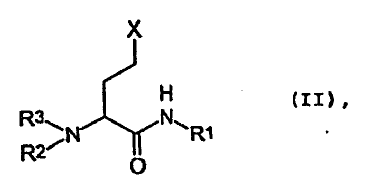 dans laquelle R¹, R² et R³ présentent la signification indiquée ci-dessus, et X représente un ester d'acide sulfonique, sont cyclisés pour donner lieu à des composés de formule générale I.

2. Procédé selon la revendication 1,
**caractérisé en ce que**
la cyclisation est réalisée dans des conditions basiques.

3. Procédé selon la revendication 2,
**caractérisé en ce**
**qu'**on utilise une solution d'hydroxyde de sodium en tant que base.

4. Procédé selon l'une ou plusieurs des revendications 1 à 3,
**caractérisé en ce que**
X représente un groupe ester d'acide sulfonique.

5. Procédé selon la revendication 4,
**caractérisé en ce que**
X représente un groupe OSO₃Me.

6. Procédé selon l'une ou plusieurs des revendications 1 à 5,
**caractérisé en ce**
**qu'**on utilise, en tant que composé II, un composé dans lequel R¹ représente un groupe p-cyanophényle ou un groupe p-carbamoylphényle, R² représente un atome d'hydrogène, et R³ représente un groupe benzyloxycarbonyle.

7. Procédé selon l'une ou plusieurs des revendications 1 à 6,
**caractérisé en ce que**
la cyclisation est réalisée à des températures de -20 à 100°C.

8. Procédé selon l'une ou plusieurs des revendications 1 à 7,
**caractérisé en ce que**
le composé de formule générale II est cyclisé sans isolation préalable pour donner lieu au composé de formule générale I.

9. Procédé selon l'une ou plusieurs des revendications 1 à 8,
**caractérisé en ce que**
des composés de formule générale II sont préparés à partir de dérivés de formule générale III 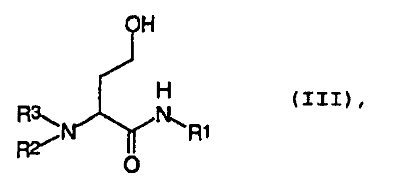 dans laquelle R¹, R² et R³ présentent la signification indiquée ci-dessus, en formant un ester d'acide sulfonique.

10. Procédé selon la revendication 9,
**caractérisé en ce que**
le composé de formule générale III ne fait pas l'objet d'une isolation intermédiaire.

11. Procédé selon l'une quelconque des revendications 9 ou 10,
**caractérisé en ce que**
le composé de formule générale III est préparé à partir de dérivés de formule générale IV 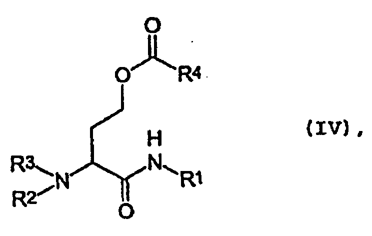 dans laquelle R¹, R² et R³ présentent la signification indiquée ci-dessus et R⁴ représente un groupe alkyle en (C₁-C₈), un groupe alcényle en (C₂-C₈), un groupe alcoxy en (C₁-C₈), un groupe alcényloxy en (C₂-C₈), qui peuvent être éventuellement linéaires ou ramifiés et éventuellement substitués par un ou plusieurs atomes d'halogène, un groupe aryle, tel qu'un groupe phényle ou naphtyle, un groupe aralkyle, tel que benzyle ou phénéthyle, un groupe arylalkyloxy, tel qu'un groupe benzyloxy.

12. Procédé selon la revendication 11,
**caractérisé en ce que**
le composé de formule IV est transformé par aminolyse en des composés de formule générale III.

13. Procédé selon la revendication 11 et/ou la revendication 12,
**caractérisé en ce que**
les composés de formule générale IV sont préparés à partir de dérivés de formule générale V 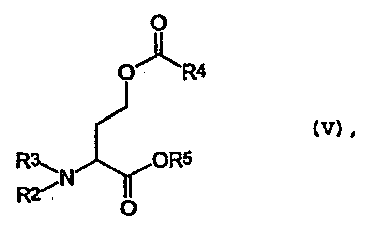 dans laquelle R² R³ et R⁴ présentent la signification indiquée ci-dessus et R⁵ représente un atome d'hydrogène, ou dans laquelle R³ et R⁵ sont liés à un noyau par l'intermédiaire d'un groupe C=O.

14. Procédé selon la revendication 13,
**caractérisé en ce que**
les composés de formule générale V, dans laquelle R⁵ représente un atome d'hydrogène, sont activés par des chlorures d'acide avant la réaction pour donner lieu à des composés de formule générale IV.

15. Procédé selon la revendication 13 et/ou la revendication 14,
**caractérisé en ce**
**qu'**un sel d'addition à un acide du composé de formule générale VI 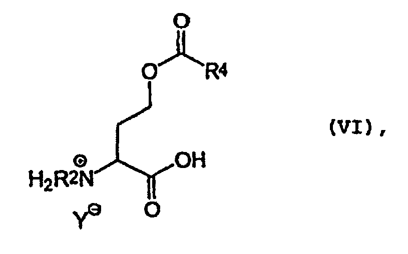 dans laquelle R² et R⁴ présentent la signification indiquée ci-dessus et Y^{⊖} est la base correspondante d'un acide inorganique, est mis à réagir avec un réactif d'acylation dérivé de R³ pour donner lieu au composé de formule générale V.

16. Procédé selon la revendication 15,
**caractérisé en ce**
**qu'**on utilise du phosgène en tant que réactif d'acylation.

17. Procédé selon la revendication 15,
**caractérisé en ce**
**qu'**on utilise un réactif d'acylation du groupe des anhydrides organiques, des esters activés et des esters d'acide halogénocarboxylique.

18. Procédé selon la revendication 17,
**caractérisé en ce que**
l'acylation est réalisée en solution aqueuse à un pH de 4 à 9.

19. Procédé selon la revendication 17,
**caractérisé en ce que**
l'acylation est réalisée dans un solvant organique en présence d'une base tertiaire.

20. Procédé selon l'une ou plusieurs des revendications 15 à 19,
**caractérisé en ce que**
le sel d'addition à un acide de formule générale VI est préparé par réaction de composés de formule générale VII 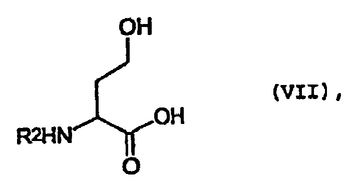 dans laquelle R² présente la signification indiquée ci-dessus, dans un mélange de R⁴COOH et de R⁴COCl ou de R⁴COBr, dans lesquels R⁴ présente la signification indiquée ci-dessus.

21. Procédé selon la revendication 20,
**caractérisé en ce que**
l'acide carboxylique et l'halogénure d'acide carboxylique sont d'abord mélangés et le composé de formule générale VII est ensuite ajouté à ce mélange.

22. Procédé selon l'une ou plusieurs des revendications 20 ou 21,
**caractérisé en ce**
**qu'**on travaille à une température de -20°C à 50°C.

23. Procédé selon l'une ou plusieurs des revendications 13 ou 14,
**caractérisé en ce**
**qu'**un composé de formule générale VIII 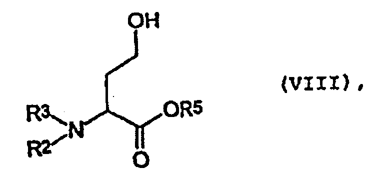 dans laquelle les radicaux R² et R³ présentent la signification indiquée ci-dessus et R⁵ représente un atome d'hydrogène, est mis à réagir avec le réactif R⁴COZ, dans lequel R⁴ présente la signification indiquée ci-dessus et Z représente un radical activé, pour donner lieu au composé de formule générale V.

24. Procédé selon la revendication 23,
**caractérisé en ce que**
dans le cas où Z représente un atome de chlore ou un atome de brome, l'acide carboxylique correspondant est utilisé en tant que solvant.

25. Procédé selon la revendication 24,
**caractérisé en ce**
**qu'**on ajoute, au mélange réactionnel en tant que base, le sel sodique de l'acide carboxylique correspondant utilisé en tant que solvant.

26. Composés de formule générale V et leurs sels 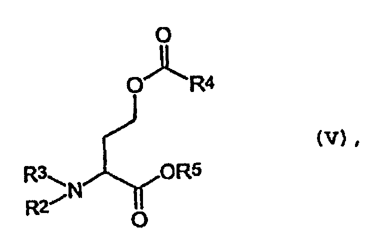 dans laquelle R² représente un atome d'hydrogène, R³ représente un groupe benzyloxycarbonyle ou un groupe tert-butyloxycarbonyle ou un groupe trifluoroacétyle, R⁴ représente un groupe méthyle et R⁵ représente un atome d'hydrogène.

27. Composés de formule générale V dans laquelle R² représente un atome d'hydrogène, R⁴ représente un groupe méthyle, et dans laquelle R³ et R⁵ sont liés à un noyau par l'intermédiaire d'un groupe C=O.

28. Composés de formule générale IV et leurs sels 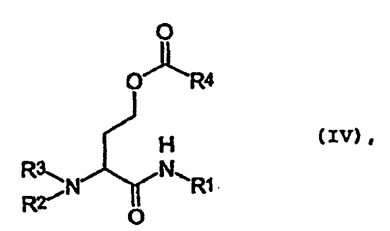 dans laquelle R¹ présente la signification selon la revendication 1, R² représente un atome d'hydrogène, R³ représente un groupe benzyloxycarbonyle ou un groupe tert-butyloxycarbonyle ou un groupe trifluoroacétyle, et R⁴ représente un groupe méthyle.

29. Composés de formule générale IV, dans laquelle R² représente un atome d'hydrogène, R³ représente un groupe benzyloxycarbonyle, R⁴ représente un groupe méthyle et R¹ représente un groupe p-cyanophényle ou un groupe p-carbamoylphényle.

30. Composés de formule générale II et leurs sels 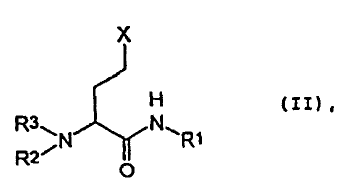 dans laquelle R¹, R² et R³ présentent la signification selon la revendication 1 et X représente un ester d'acide sulfonique.

31. Composés de formule générale II, dans laquelle R² représente un atome d'hydrogène, R³ représente un groupe benzyloxycarbonyle ou un groupe tert-butyloxycarbonyle ou un groupe trifluoroacétyle, et X représente un groupe ester d'acide sulfonique.

32. Composés de formule générale II, dans laquelle R² représente un atome d'hydrogène, R³ représente un groupe benzyloxycarbonyle, X représente un groupe OSO₃Me et R¹ représente un groupe p-cyanophényle ou un groupe p-carbamoylphényle.

33. Utilisation des composés selon les revendications 26, 29 et 32 pour la préparation d'intermédiaires pour des ingrédients bioactifs.
